(19) Europäisches Patentamt
European Patent Office
Office européen des brevets

(11) Veröffentlichungsnummer: **0 047 412**
**B1**

(12) # EUROPÄISCHE PATENTSCHRIFT

(45) Veröffentlichungstag der Patentschrift: **25.07.84**

(21) Anmeldenummer: **81106448.4**

(22) Anmeldetag: **19.08.81**

(51) Int. Cl.³: **C 07 D487/04**, C 07 D471/04, A 61 K 31/495 // (C07D487/04, 241/00, 223/00)

(54) **Neue Pyrazine, deren Herstellung und diese enthaltende Arzneimittel.**

(30) Priorität: **02.09.80 DE 3032994**

(43) Veröffentlichungstag der Anmeldung: **17.03.82 Patentblatt 82/11**

(45) Bekanntmachung des Hinweises auf die Patenterteilung: **25.07.84 Patentblatt 84/30**

(84) Benannte Vertragsstaaten: **AT BE CH DE FR IT LI LU NL SE**

(56) Entgegenhaltungen:
**EP-A- 0 003 016**
**DE-A- 2 357 253**
**DE-A- 2 519 258**
**DE-A- 2 617 101**
**US-A- 3 987 047**

(73) Patentinhaber: **Dr. Karl Thomae GmbH**
**Postfach 1755**
**D-7950 Biberach (Riss) (DE)**

(72) Erfinder: **Hurnaus, Rudolf, Dr. Dipl.-Chem.**
**Silcherstrasse 19**
**D-7950 Biberach 1 (DE)**
Erfinder: **Griss, Gerhart, Dr. Dipl.-Chem.**
**Schopperweg 1**
**D-7950 Biberach 1 (DE)**
Erfinder: **Sauter, Robert, Dr. Dipl.-Chem.**
**Albert-Schweitzer-Weg 9**
**D-7958 Laupheim (DE)**
Erfinder: **Grell, Wolfgang, Dr. Dipl.-Chem.**
**Amriswilstrasse 7**
**D-7950 Biberach 1 (DE)**
Erfinder: **Kobinger, Walter, Prof. Dr.**
**Beighofergasse 27**
**A-1121 Wien (AT)**
Erfinder: **Pichler, Ludwig, Dr.**
**Gusshausstrasse 24/11**
**A-1040 Wien (AT)**

**EP 0 047 412 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung sind neue Pyrazine der allgemeinen Formel

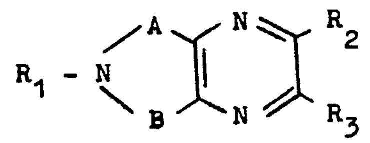

(I)

deren Säureadditionssalze, insbesondere deren physiologisch verträgliche Salze mit anorganischen oder organischen Säuren, und Verfahren zu ihrer Herstellung sowie die neuen Pyrazine enthaltende Arzneimittel.

Die neuen Verbindungen weisen wertvolle pharmakologische Eigenschaften auf, insbesondere eine herzfrequenzsenkende Wirkung.

In der obigen allgemeinen Formel I bedeutet

A und B, die gleich oder verschieden sein können, jeweils eine gegebenenfalls durch eine Alkylgruppe mit 1 bis 3 Kohlenstoffatomen substituierte Methylen- oder Äthylengruppe,

$R_1$ ein Wasserstoffatom, eine Alkylgruppe mit 4 bis 6 Kohlenstoffatomen, eine Cycloalkylgruppe mit 3 bis 7 Kohlenstoffatomen, eine Alkenylgruppe mit 3 bis 6 Kohlenstoffatomen, eine Alkyl- oder Alkanoylgruppe mit jeweils 1 bis 3 Kohlenstoffatomen, welche jeweils durch eine gegebenenfalls durch ein Halogenatom substituierte Phenylgruppe substituiert sein kann, eine Alkoxycarbonylgruppe mit insgesamt 2 bis 4 Kohlenstoffatomen, eine Aralkoxycarbonylgruppe mit insgesamt 8 bis 10 Kohlenstoffatomen oder die Phenylgruppe,

einer der Reste $R_2$ oder $R_3$ eine Aminogruppe und

der andere der Reste $R_2$ oder $R_3$ ein Wasserstoff-, Chlor- oder Bromatom, eine Alkylgruppe mit 1 bis 3 Kohlenstoffatomen, eine Alkoxygruppe mit 1 bis 3 Kohlenstoffatomen, eine Alkoxycarbonylgruppe mit insgesamt 2 bis 4 Kohlenstoffatomen, eine Phenyl- oder Halogenphenylgruppe, insbesondere jedoch

A und B je eine Äthylengruppe,

$R_1$ ein Wasserstoffatom, eine Alkylgruppe mit 1 bis 6 Kohlenstoffatomen, eine Alkenylgruppe mit 3 bis 6 Kohlenstoffatomen, eine Alkylgruppe mit 1 bis 3 Kohlenstoffatomen, welche durch eine gegebenenfalls durch ein Halogenatom substituierte Phenylgruppe substituiert ist, eine Cycloalkylgruppe mit 5 bis 7 Kohlenstoffatomen, eine Alkanoylgruppe mit 1 bis 3 Kohlenstoffatomen, eine Alkoxycarbonylgruppe mit insgesamt 2 bis 4 Kohlenstoffatomen, eine gegebenenfalls durch ein Halogenatom substituierte Benzoylgruppe oder die Phenylgruppe,

$R_2$ eine Aminogruppe und

$R_3$ ein Wasserstoffatom.

Für die bei der Definition der Reste A, B und $R_1$ bis $R_3$ eingangs erwähnten Bedeutungen kommt beispielsweise

für A und B jeweils die Bedeutung der Methylen-, Methylmethylen-, Äthylmethylen-, Propylmethylen-, Isopropylmethylen-, Äthylen-, Methyläthylen-, Äthyläthylen-, Propyläthylen- oder Isopropyläthylengruppe,

für die des Wasserstoffatoms, der Methyl-, Äthyl-, Propyl-, Isopropyl-, Butyl-, Isobutyl-, Butyl-(2)-, tert.Butyl-, Pentyl-, Isopentyl-, Neopentyl-, tert.Pentyl-, Hexyl-, Allyl-, Crotonyl-, Penten-(2)-yl-, Hexen-(2)-yl-, Cyclopropyl-, Cyclobutyl-, Cyclopentyl-, Cyclohexyl-, Cycloheptyl-, Benzyl-, Fluorbenzyl-, Chlorbenzyl-, Brombenzyl-, 1-Phenyläthyl-, 1-(Fluorphenyl)-äthyl-, 1-(Chlorphenyl)- äthyl-, 1-(Bromphenyl)-äthyl-, 1-(Chlorphenyl)-äthyl-, 1-(Bromphenyl)-äthyl-, 2-Phenyläthyl-, 2-(Fluorphenyl)-äthyl-, 2-(Chlorphenyl)-äthyl-, 2-(Bromphenyl)-äthyl-, 1-Phenylpropyl-, 2-Phenylpropyl-, 3-Phenylpropyl-, 1-(Fluorphenyl)-propyl-, 1-(Chlorphenyl)-propyl-, 2-(Chlorphenyl)-propyl-, 2-(Bromphenyl)-propyl-, 3-(Fluorphenyl)-propyl-, 3-(Chlorphenyl)-propyl-, 3-(Bromphenyl)-propyl-, Formyl-, Benzoyl-, Fluorbenzoyl-, Chlorbenzoyl-, Brombenzoyl-, Acetyl-, Phenylacetyl-, Fluorphenylacetyl-, Chlorphenylacetyl-, Bromphenylacetyl-, Propionyl-, 2-Phenylpropionyl-, 3-Phenylpropionyl-, 2-(Fluorphenyl)-propionyl-, 2-(Chlorphenyl)-propionyl-, 3-(Chlorphenyl)-propionyl-, 3(Bromphenyl)-propionyl-, Methoxycarbonyl-, Äthoxycarbonyl-, Propoxycarbonyl-, Isopropoxycarbonyl-, Benzyloxycarbonyl-, Methylbenzyloxycarbonyl-, Äthylk-benzyloxycarbonyl- oder Phenylgruppe.

für einen der Reste $R_2$ oder $R_3$ die der Aminogruppe und

für den anderen der Reste $R_2$ oder $R_3$ die des Wasserstoff-, Chlor- oder Bromatoms, der Methyl-, Äthyl-, Propyl-, Isopropyl-, Methoxy-, Äthoxy-, Propoxy-, Isopropoxy Methoxycarbonyl-, Äthoxycarbonyl-, Propoxycarbonyl-, Isopropoxycarbonyl-, Phenyl-, Fluorphenyl-, Chlorphenyl- oder Bromphenylgruppe in Betracht.

Bevorzugte Verbindungen der obigen allgemeinen Formel I sind diejenigen, in denen

A und B, die gleich oder verschieden sein können, jeweils eine Methylen-, Äthylen- oder Methyläthylengruppe,

$R_1$ eine Wasserstoffatom, eine Alkylgruppe mit 1 bis 4 Kohlenstoffatomen, eine Cycloalkylgruppe mit 3 bis 6 Kohlenstoffatomen, eine Alkenylgruppe mit 3 oder 4 Kohlenstoffatomen, eine durch eine Phenyl-oder Chlorphenylgruppe substituierte Alkylgruppe mit 1 bis 3 Kohlenstoffatomen, eine gegebenenfalls durch eine Phenyl- oder Chlorphenylgruppe substituierte Formyl- oder Acetylgruppe, eine Äthoxycarbonyl- oder Phenylgruppe,

$R_2$ ein Wasserstoff-, Chlor- oder Bromatom, eine Methyl-, Äthyl-, Methoxy-, Phenyl- oder Äthoxycarbonylgruppe und

$R_3$ eine Aminogruppe bedeuten, und deren Säureadditionssalze, insbesondere deren physiologisch verträgliche Säureadditionssalze mit anorganischen und organischen Säuren.

Besonders bevorzugte Verbindungen der obigen allgemeinen Formel I sind diejenigen, in denen

A und B jeweils eine Äthylengruppe,

$R_1$ eine Methyl-, Äthyl-, Cyclopropyl-, Cyclobutyl- oder Allylgruppe,

$R_2$ ein Wasserstoffatom und

$R_3$ eine Aminogruppe bedeuten, und deren physiologisch verträgliche Säureadditionssalze mit anorganischen oder organischen Säuren.

Erfindungsgemäß erhält man die neuen Verbindungen nach folgenden Verfahren :

a) Umsetzung eines gegebenenfalls im Reaktionsgemisch gebildeten Diketons der allgemeinen Formel

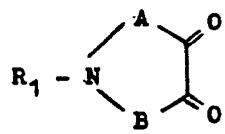

(II)

in der

A, B und $R_1$ wie eingangs definiert sind, mit einem 2-Amino-acetamidin der allgemeinen Formel

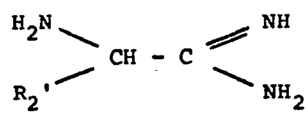

(III)

in der

$R_2'$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe bedeutet.

Die Umsetzung wird zweckmäßigerweise in einem Lösungsmittel wie Wasser, Methanol, Isopropanol, Wasser/Methanol oder Wasser/Dioxan vorzugsweise in Gegenwart einer Base wie Natriumkarbonat, Natriumhydroxid oder Pyridin, welches gleichzeitig als Lösungsmittel dienen kann, bei Temperaturen zwischen − 50 und 100 °C, vorzugsweise jedoch bei Temperaturen zwischen − 30 und 50 °C, durchgeführt.

b) Reduktion eines gegebenenfalls im Reaktionsgemisch gebildeten Pyrazin-N-oxids der allgemeinen Formel

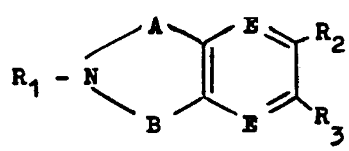

(IV)

in der

$R_1$ bis $R_3$, A und B wie eingangs definiert sind, einer der Erste E eine N-Oxidgruppe und der andere der Reste E ein Stickstoffatom oder eine N-Oxidgruppe darstellen.

Die Reduktion wird vorzugsweise in einem Lösungsmittel wie Wasser, Wasser/Äthanol, Essigsäureäthylester oder Dimethylformamid zweckmäßigerweise mit Wasserstoff in Gegenwart eines Hydrierungskatalysators wie Platin oder Palladium/Kohle, mit Metallen wie Eisen, Zinn oder Zink in Gegenwart einer Säure, mit reduzierenden Salzen wie Natriumdithionit, oder mit einem Phosphortrihalogenid wie Phosphortrichlorid bei Temperaturen zwischen 0 und 150 °C, vorzugsweise bei Temperaturen zwischen 25-100 °C, durchgeführt.

c) Cyclisierung einer gegebenenfalls im Reaktionsgemisch gebildeten Verbindung der allgemeinen Formel

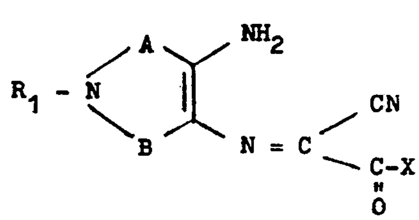

(V)

in der

$R_1$, A und B wie eingangs definiert sind und X eine Alkoxygruppe darstellt, gewünschtenfalls anschließende Hydrolyse und Decarboxylierung einer erhaltenen Verbindung der allgemeinen Formel

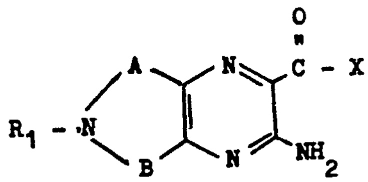

(VI)

in der

$R_1$, A und B wie eingangs definiert sind und

X wie oben definiert ist.

Die Cyclisierung wird zweckmäßigerweise in einem Lösungsmittel wie Diäthyläther, Tetrahydrofuran, Äthanol oder Dioxan vorzugsweise in Gegenwart einer Base wie einem Alkalialkoholat, z. B. Kalium-tert. butylat, bei Temperaturen zwischen − 20 und 50 °C, vorzugsweise jedoch bei Temperaturen zwischen 0 und 25 °C, durchgeführt.

Die anschließende Hydrolyse und Decarboxylierung wird in Gegenwart einer Säure wie Bromwasserstoffsäure oder Schwefelsäure, z. B. 80 %iger Schwefelsäure, bei Temperaturen zwischen 50 und 250 °C, vorzugsweise jedoch bei Temperaturen zwischen 100 und 200 °C, durchgeführt.

d) Umsetzung eines Pyrazins der allgemeinen Formel

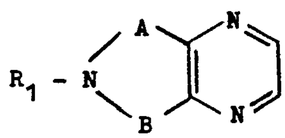

(VII)

in der

$R_1$, A und B wie eingangs definiert sind, mit einem Alkaliamid.

Die Umsetzung wird zweckmäßigerweise in einem Lösungsmittel wie Xylol, Tetralin oder Dimethylanilin, gegebenenfalls in einem Druckgefäß, vorzugsweise mit Natriumamid oder Kaliumamid bei Temperaturen zwischen 100 und 250 °C, vorzugsweise bei Temperaturen zwischen 150 und 200 °C, durchgeführt.

e) Umsetzung eines gegebenenfalls im Reaktionsgemisch gebildeten Oxims der allgemeinen Formel

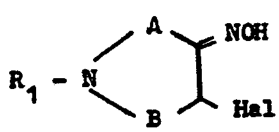

(VIII)

in der

$R_1$, A und B wie eingangs definiert sind und

Hal ein Chlor-, Brom- oder Jodatom darstellt, mit einem α-Amino-acetonitril der allgemeinen Formel

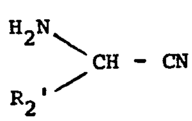

(IX)

in der

$R_2'$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe bedeutet.

Die Umsetzung wird zweckmäßigerweise in einem Lösungsmittel wie Wasser, Methanol, Wasser/Methanol, Isopropanol, Chloroform oder Dioxan vorzugsweise in Gegenwart einer Base wie Natriumcarbonat, Natriumhydroxid, Triäthylamin oder Pyridin, wobei die letzteren gleichzeitig auch als Lösungsmittel dienen können, bei Temperaturen zwischen − 50 und 100 °C, vorzugsweise jedoch bei Temperaturen zwischen − 30 und 50 °C, durchgeführt.

Eine während dieser Umsetzung im Reaktionsgemisch gebildete Verbindung der allgemeinen Formel

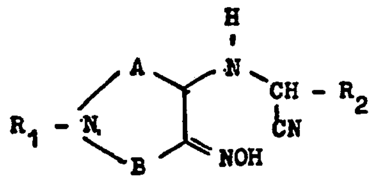

(X)

in der

$R_1$, $R_2$, A und B wie eingangs definiert sind, kann gegebenenfalls anschließend auch in Gegenwart eines sauren Kondensationsmittels wie Polyphosphorsäure, welche gleichzeitig auch als Lösungsmittel dienen kann, cyclisiert werden.

f) Umsetzung eines Pyrazins der allgemeinen Formel

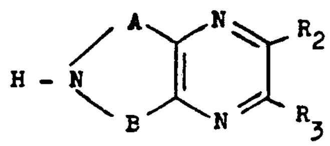

(XI)

in der

$R_2$, $R_3$, A und B wie eingangs definiert sind, mit einer Verbindung der allgemeinen Formel

$$R_1'—Y \qquad (XII)$$

in der

$R_1'$ mit Ausnahme des Wasserstoffatoms und der Phenylgruppe die für $R_1$ eingangs erwähnten Bedeutungen besitzt und

Y eine nukleophile Austrittsgruppe darstellt.

Bedeutet $R_1'$ eine der eingangs erwähnten Alkyl-, Phenylalkyl-, Cycloalkyl- oder Alkenylgruppen, so kommt beispielsweise für Y die Bedeutung eines Halogenatoms oder einer Sulfonyloxygruppe, z. B. die eines Chlor-, Brom- oder Jodatoms, die einer Methylsulfonyloxy-, Methoxysulfonyloxy- oder p-Toluolsulfonyl-oxygruppe in Betracht.

Bedeutet $R_1'$ eine der eingangs erwähnten Alkanoyl-, Phenyl-alkanoyl-, Benzoyl- oder Alkoxycarbonylgruppen, so kommt beispielsweise für die Bedeutung eines Halogenatoms, einer Aryloxygruppe oder einer Gruppe der Formel $—O—COR_1'$, z. B. die eines Chlor- oder Bromatoms, die einer Phenoxy-, Nitrophenoxy-, Acetoxy- oder Äthoxycarbonyloxygruppe, in Betracht.

Bedeutet $R_1'$ eine der eingangs erwähnten Alkylgruppen, so kommt für Y auch die Bedeutung der Hydroxygruppe in Betracht.

Die Umsetzung wird zweckmäßigerweise in einem Lösungsmittel wie Wasser, Methanol, Diäthyläther, Tetrahydrofuran, Dioxan, Acetonitril, Chloroform, Dimethylformamid oder Dimethylsulfoxid gegebenenfalls in Gegenwart eines säurebindenden Mittels wie Natriumhydroxid, Kaliumcarbonat, Kalium-tert. butylat, Triäthylamin, N-Äthyl-diisopropylamin oder Pyridin, wobei die letzteren gleichzeitig auch als Lösungsmittel dienen können, bei Temperaturen zwischen 0 und 150 °C, vorzugsweise bei Temperaturen zwischen 20 und 110 °C, durchgeführt. Die Umsetzung kann jedoch auch ohne Lösungsmittel durchgeführt werden.

Bedeutet Y die Hydroxygruppe, so wird die Umsetzung in Gegenwart von Raney-Nickel vorzugsweise bei der Siedetemperatur des zweckmäßigerweise als Lösungsmittel verwendeten Alkohols der Formel $R_1'$ OH durchgeführt.

g) Umsetzung eines Pyrazins der allgemeinen Formel

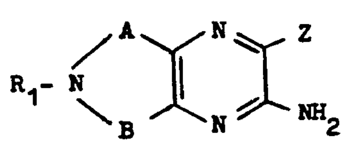

(XIII)

in der

$R_1$, A und B wie eingangs definiert sind und

Z eine nukleophile Austrittsgruppe wie ein Halogenatom, z. B. ein Chlor- oder Bromatom, darstellt, mit einer Verbindung der allgemeinen Formel

$$H—R_2'' \qquad (XIV)$$

in der

$R_2''$ eine Alkoxygruppe mit 1 bis 3 Kohlenstoffatomen darstellt, oder deren Alkalisalzen.

Die Umsetzung wird zweckmäßigerweise in einem Lösungsmittel wie Methanol, Äthanol, Dimethylformamid, Dioxan oder Dichlorbenzol, vorzugsweise jedoch in einem Überschuß der eingesetzten Verbindung der allgemeinen Formel XIV, gegebenenfalls in einem Druckgefäß und vorzugsweise in Gegenwart einer Alkalibase bei Temperaturen zwischen 50 und 200 °C, vorzugsweise jedoch bei Temperaturen zwischen 65 und 175 °C, durchgeführt.

Erhält man erfindungsgemäß eine Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, so kann diese mittels Hydrolyse und gegebenenfalls anschließender Decarboxylierung in eine entsprechende Verbindung der allgemeinen Formel I, in der $R_1$ ein Wasserstoffatom darstellt, übergeführt werden und/oder

eine Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, so kann diese mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt werden und/oder

eine Verbindung der allgemeinen Formel I, in der $R_1$ eine gegebenenfalls durch ein Halogenatom substituierte Benzylgruppe darstellt, so kann diese durch Umsetzung mit Chlorameisensäure-benzylester und anschließend mit Eisessig/Bromwasserstoff in eine entsprechende Verbindung der allgemeinen Formel I, in der $R_1$ ein Wasserstoffatom darstellt, übergeführt werden und/oder

eine Verbindung der allgemeinen Formel I, in der $R_2$ eine Wasserstoffatom und $R_3$ eine Aminogruppe darstellt, so kann diese mittels Halogenierung in eine entsprechende Verbindung der allgemeinen Formel I, in der $R_2$ ein Halogenatom darstellt, übergeführt werden.

Die nachträgliche Hydrolyse wird zweckmäßigerweise entweder in Gegenwart einer Säure wie Salzsäure, Schwefelsäure, Phosphorsäure oder Trifluoressigsäure oder in Gegenwart einer Base wie Natriumhydroxid oder Kaliumhydroxid in einem geeigneten Lösungsmittel wie Äthanol, Wasser/Äthanol, Wasser/Isopropanol oder Wasser/Dioxan bei erhöhten Temperaturen, z. B. bei der Siedetemperatur des Reaktionsgemisches, oder auch ohne Lösungsmittel durchgeführt.

Die gegebenenfalls anschließende Decarboxylierung wird durch Erhitzen zweckmäßigerweise des erhaltenen Alkalisalzes oder durch Erhitzen in einer starken Säure, z. B. in konzentrierter Salzsäure, auf Temperaturen zwischen 80 und 250 °C, vorzugsweise jedoch zwischen 100 bis 200 °C, durchgeführt.

Die nachträgliche Reduktion wird vorzugsweise mit einem komplexen Metallhydrid wie Lithiumaluminiumhydrid in einem geeigneten Lösungsmittel wie Diäthyläther, Tetrahydrofuran oder Dioxan bei Temperaturen zwischen 0 und 100 °C, vorzugsweise jedoch bei Raumtemperatur durchgeführt.

Die nachträgliche Umsetzung mit einem Chlorameisensäurebenzylester wird vorzugsweise in einem Lösungsmittel wie Chloroform, Methylenchlorid, Benzol, gegebenenfalls unter Zusatz von Triäthylamin, Pyridin oder N-Äthyl-diisopropylamin zweckmäßigerweise bei Temperaturen zwischen 0 und 50 °C und die anschließende Abspaltung des eingeführten Kohlensäureesterrestes vorzugsweise durch Erhitzen in Eisessig/Bromwasserstoff, z. B. auf 100 °C, durchgeführt.

Die nachträgliche Halogenierung wird mit einem Halogenierungsmittel wie Chlor oder Brom vorzugsweise in einem Lösungsmittel wie Eisessig oder Chloroform gegebenenfalls unter Zusatz von Pyridin bei Temperaturen zwischen 0 und 50 °C, vorzugsweise jedoch bei Raumtemperatur, durchgeführt.

Die erhaltenen Verbindungen der allgemeinen Formel I lassen sich ferner in ihre physiologisch verträglichen Salze mit anorganischen oder organischen Säuren überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure in Betracht.

Die als Ausgangsstoffe verwendeten Verbindungen allgemeinen Formeln II bis XIV sind teilweise literaturbekannt bzw. man erhält diese nach an sich bekannten Verfahren.

So erhält man beispielsweise ein Diketon der allgemeinen Formel II durch Umsetzung eines entsprechenden Tetrahydro-bis-(tri-alkylsilyloxy)-azepins in einem Lösungsmittel wie Methanol, Tetrachlorkohlenstoff, Pyridin und/oder Dioxan mit Brom, Kupfer-II-acetat oder Luftsauerstoff zwischen 0 und 100 °C oder durch Selendioxid-Oxidation eines entsprechenden Hexahydro-azepinons.

Eine als Ausgangsstoff verwendete Verbindung der allgemeinen Formel IV erhält man beispielsweise durch Umsetzung eines entsprechenden Oxims der allgemeinen Formel

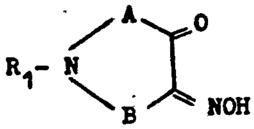

(XV)

in der

$R_1$, A und B wie eingangs definiert sind, mit einem entsprechenden Amino-acetonitril in Gegenwart

einer Base wie Triäthylamin, Pyridin oder N-Äthyl-diisopropylamin bei Temperaturen zwischen 0 und 100 °C, vorzugsweise jedoch bei Temperaturen zwischen Raumtemperatur und der Siedetemperatur des Reaktionsgemisches. Die hierfür erforderliche Verbindung der allgemeinen Formel XV erhält man durch Umsetzung eines entsprechenden Azepinons mit einem Alkylnitrit, z. B. mit tert.Butylnitrit, in einem Lösungsmittel wie Diäthyläther, Tetrahydrofuran oder Dioxan bei Temperaturen zwischen – 50 und 25 °C. Die hierfür benötigten Azepinone sind literaturbekannt (siehe A. K. Jokoo et al. Bull. Chem. Soc. Japan 29, 631 (1959) und US-PS 3 987 047) bzw. die hierfür benötigten Tetrahydro-4,5-bis(trimethylsilyloxy)-azepine erhält man durch Acyloin-Kondensation der entsprechenden Dicarbonsäurediester mit Natrium in Gegenwart von Chlor-trimethylsilan (siehe J. Org. Chem. 42, 24 (1977) oder Synthesis 236, (1971)).

Eine als Ausgangsstoff verwendete Verbindung der allgemeinen Formel V erhält man durch Umsetzung eines entsprechenden Enamins, welches man beispielsweise durch Umsetzung eines entsprechenden Azepinons mit Morpholin erhält, mit einem o-(p-Toluolsulfonyl)-isonitrosocyanessigsäure-äthylester und anschließend mit Ammoniak.

Eine als Ausgangsstoff verwendete Verbindung der allgemeinen Formel VIII erhält man beispielsweise durch Halogenierung eines entsprechenden Azepinons, durch Umsetzung des so erhaltenen Hydrogenhalogenids mit jeweils einem Äquivalent Hydroxylamin-hydrochlorid und Natriumäthylat, wobei das Hydrohalogenid einer Verbindung der allgemeinen Formel VIII erhalten wird.

Eine als Ausgangsstoff verwendete Verbindung der allgemeinen Formel XI oder XIII erhält man durch Kondensation eines entsprechenden Diketons mit einem entsprechenden 2-Amino-acetamidin und gegebenenfalls anschließende Hydrolyse und/oder Halogenierung.

Wie bereits eingangs erwähnt, weisen die neuen Verbindungen der allgemeinen Formel I und deren physiologisch verträgliche Säureadditionssalze wertvolle pharmakologische Eigenschaften auf, insbesondere neben einer Beeinflussung des Blutdrucks eine starke herzfrequenzsenkende Wirkung.

Beispielsweise wurden die Verbindungen

A = 7-äthyl-2-amino-6,7,8,9-tetrahydro-5H-Pyrazino[2,3-d]azepin-dihydrochlorid,
B = 7-Allyl-2-amino-6,7,8,9-tetrahydro-5H-Pyrazino[2,3-d]azepin-dihydrochlorid,
C = 2-Amino-6,7,8,9-tetrahydro-7-methyl-5H-Pyrazino[2,3-d]azepin-dihydrochlorid,
D = 7-Äthyl-2-amino-3-brom-6,7,8,9-tetrahydro-5H-pyrazino[2,3-d]-azepin-hydrochlorid,
E = 7-Äthyl-2-amino-3-chlor-6,7,8,9-tetrahydro-5H-Pyrazino-[2,3-d]azepin-hydrochlorid,
F = 7-Äthyl-2-amino-6,7,8,9-tetrahydro-3-methyl-5H-pyrazino-[2,3-d]azepin-dihydrochlorid,
G = 6-Äthyl-2-amino-5,6,7,8-tetrahydro-pyrido[3,4-b]pyrazin-dihydrochlorid

und

H = 2-Amino-7-cyclopropyl-6,7,8,9-tetrahydro-5H-pyrazino[2,3-d]-azepin-hydrochlorid

auf ihre biologischen Eigenschaften wie folgt untersucht :

1. Wirkung auf Blutdruck und Herzfrequenz

Methodik :

An der intakten, narkotisierten Ratte werden die Kreislaufveränderungen nach intravenöser Injektion der Testsubstanzen registriert.

Versuchsbeschreibung :

Männliche Ratten im Gewicht von 120-360 g werden mit Pentobarbital (70 mg/kg) narkotisiert. Die Tiere atmen spontan über eine eingebundene Trachealkanüle. Die Blutdruckmessung erfolgt aus einer A. carotis mit Hilfe eines elektro-mechanischen Druckwandlers ; die Pulswelle triggert einen Tachographen, der kontinuierlich die Herzfrequenz registriert. Beide Parameter werden auf einem Polygraphen aufgezeichnet. Testsubstanzen werden über einen Katheter in eine V. jugularis injiziert.

Die zu untersuchenden Substanzen führten mit 3 mg/kg i.v. nach kurzdauernder initialer Blutdrucksteigerung zu längerdauernder Drucksenkung. Die Herzfrequenz war ausgeprägt und langdauernd gesenkt. Die maximalen Werte sind aus der nachstehenden Tabelle zu entnehmen :

(Siehe Tabelle, Seite 8 f.)

| Substanz | Dosis mg/kg i.v. | Blutdruck in mm Hg pressorische Phase | depressorische Phase | Herzfrequenzsenkung Schläge/Minute |
|---|---|---|---|---|
| A | 3 | + 61 | - 20 | - 116 |
| B | 3 | + 40 | - 20 | - 122 |
| C | 3 | + 26 | - 36 | - 105 |
| D | 3 | + 50 | - 5 | - 55 |
| E | 3 | + 32 | - 11 | - 70 |
| F | 3 | + 38 | - 10 | - 78 |
| G | 3 | + 6 | - 41 | - 145 |
| H | 3 | + 51 | - 28 | - 110 |

2. Wirkung an praesynaptischen α-Adrenoceptoren : Hemmung der elektrisch ausgelösten Tachycardie

Methodik :

Am adrenergenen Nervenende existiert ein Autoregulationssystem der Noradrenalinfreisetzung. Erregung praesynaptischer α-Adrenorezeptoren wirkt hemmend auf die Noradrenalinfreisetzung und damit einschränkend auf die adrenergene Neurotransmission. Dementsprechend wird bei Stimulierung dieser « feedback Rezeptoren » der Effekt einer gegebenen adrenergen Reizung am Erfolgsorgan abgeschwächt werden.

Am Modell der elektrisch gereizten Spinalratte wird die am Herzen elektrisch induzierte Tachykardie durch praesynaptisch α-mimetisch wirksame Substanzen abgeschwächt.

Versuchsbeschreibung :

Männliche Ratten im Gewicht von 350-400 g werden mit Pentobarbital (50 mg/kg i. p.) narkotisiert, vagotomiert, mit Atropin (1 mg/kg s. c.) behandelt und trachealkanüliert. Nach Enukleation eines Auges wird die Orbita durchstoßen, das Gehirn zerstört und ein Metallstab durch den Wirbelkanal vorgeschoben. Die Tiere werden künstlich beatmet und mit Gallamin (4 mg/kg i. v.) vorbehandelt. Der verwendete Metallstab hat einen Durchmesser von 2,5 mm und ist isoliert (einbrennemailliert). Vom 7.-8. cm von der SPitze gemessen ist der Stab balnk. Dieses blanke Stück kommt beim Vorschieben des Stabes auf Höhe des letzten Hals- und ersten Brustwirbels zu liegen. Gegen eine Gegenelektrode, die in die Nackenhaut der Tiere eingestochen wird, wird mittels eines Stimulators supramaximal gereizt (Rechteckimpulse 50V, 2 ms ; 0,2 Hz ; Reizdauer 25 s). Der Blutdruck wird aus einer A. carotids mittels elektromechanischem Druckwandler gemessen. Die Pulswelle triggert einen Tachographen, der kontinuierlich die Herzfrequenz wiedergibt. Beide SIgnale werden auf einem Mehrkanalschreiber registriert.

Es wird die Dosis ($D_{50}$) angegeben, welche die elektrisch induzierte Tachykardie um 50 % hemmt.

| Substanz | Hemmung der elektrisch ausgelösten Tachykardie $D_{50}$ |
|---|---|
| A | 28 µg/kg i.v. |
| G | 670 µg/kg i.v. |

3. Wirkung an postsynaptischen α-Adrenoceptoren : Blutdrucksteigerung an der Spinalratte :

Methodik :

Am Modell der Spinalratte wird die nach Erregung postsynaptischer α-Adrenorezeptoren eintretende Blutdrucksteigerung gemessen.

Versuchsbeschreibung :

Männliche Ratten im Gewicht von 200-250 g werden narkotisiert (1,2 g/kg Urethan i. p., 1 mg/kg Atropin s. c.).

Dann erfolgt Eröffnung des Rückenmarkkanals. Anschließend werden bei $C_1$ das Rückenmark durchtrennt und die Medulla oblongata und Gehirn mit einer Metallsonde zerstört. Die Tiere werden künstlich beatmet; in eine Jugularvene wird ein Polyäthylen-Katheter eingebunden, die Registrierung des Blutdrucks erfolgt aus einer A. carotis mittels Statham-Element an einem Grass-Polygraphen.

Es wird die Dosis ($D_{30}$) angegeben, welche den arteriellen Blutdruck um 30 mmHg steigert.

| Substanz | Blutdrucksteigernde Wirkung $D_{30}$ |
|---|---|
| A | 480 µg/kg i.v. |
| B | 2,6 mg/kg i.v. |
| G | 1,4 mg/kg i.v. |

4. Akute Toxizität:

Die akute Toxizität der zu untersuchenden Substanzen wurde an Mäusen nach oraler bzw. intravenöser Gabe bestimmt (Beobachtungszeit: 14 Tage):

| Substanz | $LD_{50}$ |
|---|---|
| A | 215 mg/kg i.v.<br>1 250 mg/kg p.o. |

Auf Grund ihrer pharmakologischen Eigenschaften eignen sich die neuen Verbindungen der allgemeinen Formel I sowie deren physiologisch verträgliche Säureadditionssalze zur Behandlung von Herz- und Kreislauferkrankungen, pectanginösen Beschwerden sowie zur Senkung der Herzfrequenz. Hierzu lassen sich diese, gegebenenfalls in Kombination mit anderen Wirksubstanzen, in die üblichen galenischen Zubereitungsformen wie Dragées, Tabletten, Kapseln, Suppositorien oder Lösungen einarbeiten. Die Einzeldosis beträgt hierbei am Erwachsenen 0,2-10 mg vorzugsweise 0,2-5 mg 1 bis 3 x täglich.

Die nachfolgenden Beispiele sollen die Erfindung näher erläutern:

## Beispiel A

7-Äthyl-2-amino-6,7,8,9-tetrahydro-5H-pyrazino [2,3-d] azepin-1-oxid-dihydrochlorid

19 g (0,16 Mol) Kalium-tert. butylat werden in 200 ml absolutem Tetrahydrofuran vorgelegt. Bei – 40 °C gibt man 23 g (0,16 Mol) 1-Äthyl-hexahydro-azepinon-4 und anschließend 19,2 ml tert.-Butylnitrit tropfenweise zu. Nach Stehen über Nacht bei 0-10 °C fügt man eine Lösung von 20 g (0,217 Mol) Aminoacetonitril-hydrochlorid und 30 g N-Äthyl-diisopropylamin in 200 ml Chloroform zu. Dann kocht man 4 Stunden am Rückfluß, filtriert, engt ein und isoliert aus dem angefallenen Isomerengemisch durch Kieselgelchromatographie mit Aceton/konz. Ammoniak 95 : 5 als Fließmittel die gewünschte Verbindung. Anschließend wird in Äthanol das Dihydrochlorid mit äthanolischer Salzsäure gefällt.

Ausbeute: 1,5 g (3,3 % der Theorie),
Schmelzpunkt; 227 °C (Zers.).

| | | | | |
|---|---|---|---|---|
| Ber: | C 42,71 | H 6,45 | N 19,92 | Cl 25,22 |
| Gef.: | 42,60 | 6,68 | 19,63 | 24,60 |

## Beispiel B

2-Amino-7-benzyl-6,7,8,9-tetrahydro-5H-pyrazino [2,3-d] azepin-1-oxid

Hergestellt aus 1-Benzyl-hexahydro-azepinon-4 durch Oximierung mit tert. Butylnitrit und anschließende Umsetzung mit Amino-acetonitril analog Beispiel A.

Ausbeute: 4,6 % der Theorie,
Schmelzpunkt: < 20 °C.
Ber.: Molpeak m/e = 270
Gef.: Molpeak m/e = 270

Beispiel C

7-Acetyl-2-amino-6,7,8,9-tetrahydro-5H-pyrazino [2,3-d] azepin-1-oxid

Hergestellt analog Beispiel A aus 1-Acetyl-hexahydro-azepin-on-4 durch Oximierung mit tert. Butylnitrit und anschließende Umsetzung mit Aminoacetonitril.

Ausbeute : 9,7 % der Theorie,
Schmelzpunkt : < 20 °C.
Ber. : Molpeak m/e = 222
Gef. : Molpeak m/e = 222

Beispiel D

2-Amino-6,7,8,9-tetrahydro-5H-7-pyrazino [2,3-d] azepincarbonsäure-äthylester-1-oxid

Hergestellt analog Beispiel A aus Hexahydro-azepin-4-on-1-carbonsäure-äthylester durch Oximierung mit tert. Butylnitrit und anschließende Umsetzung mit Aminoacetonitril.

Ausbeute : 11,8 % der Theorie,
Schmelzpunkt : < 20 °C.
Ber. : Molpeak m/e = 252
Gef. : Molpeak m/e = 252

Beispiel 1

7-Äthyl-2-amino-6,7,8,9-tetrahydro-5H-Pyrazino [2,3-d] azepin-dihydrochlorid

7,5 g (25 mMol) 1-Äthyl-2,3,6,7-tetrahydro-4,5-bis(trimethylsilyloxy)-azepin werden in 100 ml absolutem Dioxan gelöst und unter Kühlung mit einer Lösung von 4 g (25 mMol) Brom in 50 ml absolutem Dioxan tropfenweise versetzt. Die so erhaltene Reaktionsmischung wird nach Zugabe von 8 ml absolutem Pyridin unter Eiskühlung und kräftigem Rühren zu einer Lösung von 4,8 g (40 mMol) 2-Amino-acetamidin-dihydrochlorid in 200 ml Wasser getropft. Man rührt noch 5 Stunden bei Raumtemperatur nach, engt dann ein, stellt mit 2N Natronlauge stark alkalisch und extrahiert mit Chloroform. Nach dem Trocknen der Extrakte über Natriumsulfat wird eingeengt und mit Petroläther digeriert. Das nach Abdekantieren zurückbleibende Öl wird in Äthanol gelöst und als Dihydrochlorid gefällt.

Ausbeute : 3,70 g (56 % der Theorie),
Schmelzpunkt : 236 °C (Zers.).

| | | | | |
|---|---|---|---|---|
| Ber. : | C 45,29 | H 6,84 | N 21,13 | Cl 26,74 |
| Gef. : | 45,61 | 6,96 | 21,47 | 26,45 |

Beispiel 2

2-Amino-6,7,8,9-tetrahydro-7-methyl-5H-pyrazino [2,3-d] azepin-dihydrochlorid

Hergestellt analog Beispiel 1 aus 2,3,6,7-Tetrahydro-1-methyl-4,5-bis(trimethylsilyloxy)-azepin und 2-Amino-acetamidin-dihydro-bromid.

Ausbeute : 45 % der Theorie,
Schmelzpunkt : 224 °C (Zers.).

| | | | | |
|---|---|---|---|---|
| Ber. : | C 43,04 | H 6,42 | N 22,31 | Cl 28,23 |
| Gef. : | 43,51 | 6,51 | 21,30 | 27,95 |

Beispiel 3

2-Amino-7-benzyl-6,7,8,9-tetrahydro-5H-pyrazino [2,3-d] azepin

70 g (0,193 Mol) 1-Benzyl-2,3,6,7-tetrahydro-4,5-bis(trimethylsilyloxy)-azepin werden in 900 ml Tetrachlorkohlenstoff gelöst und unter Rühren und Kühlung mit 28,8 g (0,18 Mol) Brom in 80 ml Tetrachlorkohlenstoff tropfenweise versetzt. Nach dem Einengen wird in 400 ml Methanol aufgenommen, mit 100 g Eis versetzt und 41,1 g (0,175 Mol) 2-Amino-acetamidin-dihydrobromid zugefügt. Dann tropft man bei 0-5 °C langsam 2N Natronlauge zu. Sobald ein konstanter pH-Wert von 5 erreicht ist, läßt man 24 Stunden im Kühlschrank stehen. Danach wird eingeengt, in Wasser aufgenommen, filtriert und mit Natronlauge alkalisch gestellt. Man saugt den ausgefallenen gelbbraunen Kristallbrei ab und trocknet im Vakuum.

Ausbeute : 9,1 g (20,4 % der Theorie),
Schmelzpunkt : 112 °C.

Aus dem wässrigen Filtrat werden durch Extraktion mit Methylenchlorid und anschließende

Säulenreinigung an Kieselgel mit Methanol/Methylenchlorid = 3 : 1 als Fließmittel noch weitere 5,3 g (11,9 % der Theorie) erhalten.

| | | | | |
|---|---|---|---|---|
| Ber. : | C 70,84 | H 7,13 | N 22,03 | |
| Gef. : | 70,55 | 7,05 | 21,98 | |

Beispiel 4

2-Amino-6,7,8,9-tetrahydro-7-isopropyl-5H-pyrazino [2,3-d] azepin-dihydrochlorid

Hergestellt analog Beispiel 1 aus 2,3,6,7-Tetrahydro-1-isopropyl-4,5-bis(trimethylsilyloxy)-azepin und 2-Amino-acetamidin-dihydrobromid.
Ausbeute : 46 % der theorie,
Schmelzpunkt : 222-224 °C.

| | | | | |
|---|---|---|---|---|
| Ber. : | C 47,32 | H 7,22 | N 20,07 | Cl 25,40 |
| Gef. : | 47,32 | 7,65 | 19,88 | 25,30 |

Beispiel 5

2-Amino-6,7,8,9-tetrahydro-7-propyl-5H-pyrazino [2,3-d] azepin

Hergestellt analog Beispiel 1 aus 2,3,6,7-Tetrahydro-1-propyl-4,5-bis(trimethylsilyloxy)-azepin und 2-Amino-acetamidin-dihydro-bromid.
Ausbeute : 20 % der Theorie,
Schmelzpunkt : 149 °C.

| | | | |
|---|---|---|---|
| Ber. : | C 64,03 | H 8,79 | N 27,16 |
| Gef. : | 63,80 | 8,75 | 27,19 |

Beispiel 6

7-Allyl-2-amino-6,7,8,9-tetrahydro-5H-pyrazino [2,3-d] azepin-dihydrochlorid

Hergestellt aus 1-Allyl-2,3,6,7-tetrahydro-4,5-bis(trimethylsilyloxy)-azepin und 2-Amino-acetamidin-dihydrobromid analog Beispiel 1.
Ausbeute : 26 % der Theorie,
Schmelzpunkt ; 272 °C (Zers.).

| | | | | |
|---|---|---|---|---|
| Ber. : | C 47,66 | H 6,55 | N 20,21 | Cl 25,58 |
| Gef. : | 47,50 | 6,39 | 20,12 | 25,55 |

Beispiel 7

2-Amino-7-butyl-6,7,8,9-tetrahydro-5H-pyrazino [2,3-d] azepin

Hergestellt aus 1-Butyl-2,3,6,7-tetrahydro-4,5-bis(trimethylsilyloxy)-azepin und 2-Amino-acetamidin-dihydrobromid analog Beispiel 3.
Ausbeute : 11,3 g (21,4 % der Theorie),
Schmelzpunkt : 140-142 °C.

| | | | |
|---|---|---|---|
| Ber. : | C 65,42 | H 9,15 | N 25,44 |
| Gef. : | 65,23 | 9,13 | 25,56 |

Beispiel 8

2-Amino-6,7,8,9-tetrahydro-7-isobutyl-5H-pyrazino [2,3-d] azepin-dihydrochlorid

Hergestellt aus 2,3,6,7-Tetrahydro-1-isobutyl-4,5-bis(trimethylsilyloxy)-azepin und 2-Amino-acetamidin-dihydrobromid analog Beispiel 3.
Ausbeute : 13 % der Theorie,
Schmelzpunkt : 149-151 °C.

| | | | | |
|---|---|---|---|---|
| Ber. : | C 49,15 | H 7,56 | N 19,11 | Cl 24,18 |
| Gef. : | 49,23 | 7,63 | 19,12 | 24,00 |

Beispiel 9

2-Amino-7-butyl-(2)-6,7,8,9-tetrahydro-5H-pyrazino [2,3-d] azepin

Hergestellt aus 1-Butyl-(2)-2,3,6,7-tetrahydro-4,5-bis(trimethylsilyloxy)-azepin und 2-Amino-acetamidin-dihydrobromid analog Beispiel 3.

Ausbeute : 15 % der Theorie,
Schmelzpunkt : 113 °C.

| | | | |
|---|---|---|---|
| Ber. : | C 65.42 | H 9,15 | N 25,43 |
| Gef. : | 65,85 | 8,99 | 25,64 |

Beispiel 10

2-Amino-7-tert.butyl-6,7,8,9-tetrahydro-5H-pyrazino [2,3-d] azepin

Hergestellt aus 1-tert.Butyl-2,3,6,7-tetrahydro-4,5-bis(trimethylsilyloxy)-azepin und 2-Amino-acetamidin-dihydrobromid analog Beispiel 3.
Ausbeute : 37 % der Theorie,
Schmelzpunkt : 128 °C.

| | | | |
|---|---|---|---|
| Ber. : | C 65,41 | H 9,14 | N 25,43 |
| Gef. : | 65,20 | 9,18 | 25,49 |

Schmelzpunkt des Dihydrochlorids : 190 °C (Zers.).

Beispiel 11

2-Amino-7-cyclohexyl-6,7,8,9-tetrahydro-5H-pyrazino [2,3-d] azepin

Hergestellt aus 1-Cyclohexyl-2,3,6,7-tetrahydro-4,5-bis(trimethylsilyloxy)-azepin und 2-Amino-acetamidin-dihydrobromid analog Beispiel 1.
Ausbeute : 35 % der Theorie,
Schmelzpunkt : 167 °C.

| | | | |
|---|---|---|---|
| Ber. : | C 68,26 | H 9,00 | N 22,74 |
| Gef. : | 68,03 | 9,24 | 22,24 |

Beispiel 12

2-Amino-6,7,8,9-tetrahydro-7-phenyl-5H-pyrazino [2,3-d] azepin

35 g (0,1 Mol) 2,3,6,7-Tetrahydro-1-phenyl-4,5-bis(trimethylsilyloxy)-azepin werden in 750 ml Methanol 2 Stunden gekocht. Nach Zusatz von 60 g (0,3 Mol) Kupfer-II-acetat wird weitere 60 Minuten gekocht. Dann wird filtriert und eingeengt. Der Einengungsrückstand wird mit Cyclohexan ausgekocht. Die Cyclohexanextrakte werden eingeengt und nach Lösen in 250 ml Methanol mit 500 g Eis und 20,6 g (0,08 Mol) 2-Amino-acetamidin-dihydrobromid versetzt. Anschließend wird 2N Natronlauge zugetropft bis ein konstanter pH-Wert von 5 erreicht wird. Nach Stehen über Nacht im Kühlschrank wird eingeengt, alkalisch gestellt und mit Methylenchlorid extrahiert. Die getrockneten und eingeengten Extrakte werden über Kieselgel mit Essigsäureäthylester als Fließmittel chromatographiert.
Ausbeute : 1,7 g (8,9 % der Theorie),
Schmelzpunkt : 95-97 °C.

| | | | |
|---|---|---|---|
| Ber. : | C 69,97 | H 6,71 | N 23,22 |
| Gef. : | 69,70 | 6,70 | 23,05 |

Beispiel 13

2-Amino-7-(4-chlor-benzoyl)-6,7,8,9-tetrahydro-5H-Pyrazino [2,3-d] azepin

53 g (0,2 Mol) eines Gemisches von 1-(4-Chlor-benzoyl)-hexahydroazepin-4,5-dion und 1-(4-Chlorbenzoyl)-hexahydro-azepin-3,4-dion [hergestellt aus 1-(4-Chlorbenzoyl)-hexahydro-azepinon-4 durch Selendioxid-Oxidation in Dioxan/Wasser] und 47 g (0,2 Mol) 2-Amino-acetamidin-dihydrobromid werden in 700 ml Methanol gelöst und bei 5 °C tropfenweise mit 200 ml 2N-Natronlauge versetzt. Man rührt eine Stunde bei Raumtemperatur nach, engt dann ein und extrahiert nach Zusatz von weiterer Natronlauge mit Chloroform. Die getrockneten Extrakte werden eingeengt und an Kieselgel in Essigsäureäthylester/Methanol 10 : 1 chromatographiert. Die erhaltenen Kristalle werden mit Essigsäureäthylester digeriert und abgesaugt.
Ausbeute : 8,8 g (14,5 % der Theorie),
Schmelzpunkt : 190-192 °C.

| | | | | |
|---|---|---|---|---|
| Ber. : | C 59,50 | H 4,99 | N 18,51 | Cl 11,71 |
| Gef. : | 59,93 | 4,70 | 18,41 | 12,23 |

Beispiel 14

7-Acetyl-2-amino-6,7,8,9-tetrahydro-5H-pyrazino [2,3-d] azepin

Erhalten durch Umsetzung eines gemisches von 1-Acetyl-hexahydro-azepin-4,5-dion und 1-Acetyl-hexahydro-azepin-3,4-dion (hergestellt aus 1-Acetyl-hexahydro-azepin-4-on durch Selendioxidoxidation in Dioxan/Wasser) mit 2-Amino-acetamidin-dihydrobromid analog Beispiel 13.

Ausbeute : 17 % der Theorie,
Schmelzpunkt : 205 °C.

| | | | |
|---|---|---|---|
| Ber. : | C 58,23 | H 6,84 | N 27,17 |
| Gef. : | 58,01 | 6,51 | 26,88 |

Beispiel 15

2-Amino-6,7,8,9-tetrahydro-5H-7-pyrazino [2,3-d] azepincarbonsäure-äthylester

Hergestellt durch Umsetzung eines Gemisches von Hexahydroazepin-3,4-dion-1-carbonsäureäthylester und Hexahydro-azepin-4,5-dion-1-carbonsäure-äthylester (erhalten aus Hexahydroazepin-4-on-1-carbonsäure-äthylester durch Selendioxid-Oxidation) mit 2-Amino-acetamidin-dihydrobromid analog Beispiel 13.

Ausbeute : 21 % der Theorie,
Schmelzpunkt : 135 °C.

| | | | |
|---|---|---|---|
| Ber. : | C 55,91 | H 6,89 | N 23,71 |
| Gef. : | 56,18 | 6,87 | 23,40 |

Beispiel 16

2-Amino-6,7,8,9-tetrahydro-5H-Pyrazino [2,3-d] azepin

11,15 g (47,2 mMol) 2-Amino-6,7,8,9-tetrahydro-5H-7-pyrazino [2,3-d] azepincarbonsäure-äthylester werden in 300 ml Äthanol gelöst und mit 30 g Kaliumhydroxid versetzt. Die Lösung wird zur Trockne eingedampft, und der Rückstand 3 Stunden auf 100 °C erhitzt. Anschließend wird mit Wasser aufgenommen und mit Hilfe eines Schwerphasenperforators mit Chloroform extrahiert. Der Chloroformextrakt wird über eine Kieselgelsäule mit Chloroform/Methanol/konz. Ammoniak 24 : 12 : 1 als Fließmittel gereinigt.

Ausbeute : 3,6 g (46 % der Theorie),
Schmelzpunkt : 138-140 °c.

| | | | |
|---|---|---|---|
| Ber. : | C 58,51 | H 7,37 | N 34,11 |
| Gef. : | 58,68 | 7,28 | 33,95 |

Beispiel 17

2-Amino-7-(4-chlorbenzyl)-6,7,8,9-tetrahydro-5H-pyrazino [2,3-d] azepin

3,7 g (12,2 mMol) 2-Amino-7-(4-chlor-benzoyl)-6,7,8,9-tetrahydro-5H-pyrazino [2,3-d] azepin werden in 50 ml absolutem Tetrahydrofuran gelöst und tropfenweise einer Suspension von 2,8 g (73,3 mMol) Lithiumaluminiumhydrid zugefügt. Es wird 3 Stunden bei Raumtemperatur nachgerührt, mit 2N Natronlauge überschüssiges Lithiumaluminiumhydrid zersetzt und vom Aluminat abfiltriert. Nach dem Einengen des Filtrats wird an Kieselgel mit Essigsäureäthylester/Methanol = 5 : 1 als Fließmittel chromatographiert. Anschließend wird in absolutem Äther gelöst und bis zur beginnenden Kristallisation eingeengt.

Ausbeute : 1,5 g (43 % der Theorie),
Schmelzpunkt : 158 °C.

| | | | |
|---|---|---|---|
| Ber. : | C 62,38 | H 5,94 | N 19,41 |
| Gef. : | 62,27 | 6,10 | 18,94 |

Beispiel 18

7-Äthyl-2-amino-6,7,8,9-tetrahydro-5H-pyrazino [2,3-d] azepin-monohydrochlorid

Hergestellt aus 7-Acetyl-2-amino-6,7,8,9-tetrahydro-5H-pyrazino [2,3-d] azepin durch Reduktion mit Lithiumaluminiumhydrid analog Beispiel 17 und anschließende Fällung als Monohydrochlorid.

Ausbeute : 28 % der Theorie,
Schmelzpunkt : 247-251 °C (Zers.).

| | | | | |
|---|---|---|---|---|
| Ber. : | C 52,51 | H 7,49 | N 24,50 | Cl 15,50 |
| Gef. : | 52,61 | 7,67 | 24,30 | 15,15 |

Beispiel 19

7-Äthyl-2-amino-6,7,8,9-tetrahydro-5H-pyrazino [2,3-d] azepin-dihydrochlorid

1,04 g (5 mMol) 7-Äthyl-2-amino-6,7,8,9-tetrahydro-5H-pyrazino [2,3-d] azepin-1-oxid werden in 15 ml Wasser und 15 ml Äthanol gelöst und nach Zugabe von 2 g Natriumdithionit 30 Stunden am Rückfluß gekocht, wobei jede Stunde weitere 0,5 Natriumdithionit in je 5 ml Wasser und 5 ml Äthanol zugefügt werden. Dann wird eingeengt, alkalisch gestellt und mit Chloroform extrahiert. Nach Kieselgelchromatographie wird in Äthanol mit äthanolischer Salzsäure das Hydrochlorid gefällt.

Ausbeute : 0,45 g (34 % der Theorie),
Schmelzpunkt : 233 °C (Zers.).

Beispiel 20

2-Amino-6,7,8,9-tetrahydro-5H-Pyrazino [2,3-d] azepin

a) 2-Amino-6,7,8,9-tetrahydro-5H-3,7-pyrazino [2,3-d] azepin-dicarbonsäurediäthylester

25,4 g (0,1 Mol) eines Isomerengemisches von 4-Morpholino-2,5,6,7-tetrahydro-1H-azepin-1-carbonsäureäthylester und 4-Morpholino-2,3,6,7-tetrahydro-1H-azepin-1-carbonsäureäthylester werden bei Raumtemperatur zu einer Lösung von 14,8 g (50 mMol) o-(p-Toluolsulfonyl)-isonitrosocyanessigsäureäthylester in 250 ml absolutem Äther getropft. Nach Stehen über Nacht wird eingeengt, in Methylenchlorid aufgenommen und mit Wasser gewaschen. Nach Trocknung über Natriumsulfat wird der Methylenchloridextrakt eingeengt. Den Rückstand versetzt man mit 300 ml gesättigter äthanolischer Ammoniaklösung und läßt 24 Stunden stehen. Nach dem Einengen wird an Kieselgel mit Essigester als Fließmittel chromatographiert. Nach Digerieren mit wenig Äther wird abgesaugt und aus Acetonitril umkristallisiert.

Ausbeute : 1,6 g (10 % der Theorie),
Schmelzpunkt : 166-168 °C.

| | | | |
|---|---|---|---|
| Ber. : | C 54,53 | H 6,54 | N 18,17 |
| Gef. : | 54,69 | 6,43 | 18,55 |

b) 2-Amino-6,7,8,9-tetrahydro-5H-pyrazino [2,3-d] azepin

1,6 g (5,2 mMol) 2-Amino-6,7,8,9-tetrahydro-5H-3,7-pyrazino [2,3-d] azepindicarbonsäure-diäthylester werden mit 20 ml 80 %iger Schwefelsäure 30 Minuten auf 180 °C erhitzt. Dann wird abgekühlt, mit Sodalösung alkalisch gestellt und mit Chloroform mittels eines Perforators 24 Stunden extrahiert. Nach dem Trocknen des Chloroformextraktes wird an Kieselgel chromatographiert mit Chloroform/Methanol/konz. Ammoniak = 24 : 12 : 1 als Fließmittel.

Ausbeute : 0,1 g (11,7 % der Theorie),
Schmelzpunkt : 137-139 °c.

Beispiel 21

2-Amino-7-benzyl-6,7,8,9-tetrahydro-5H-pyrazino [2,3-d] azepin

a) 7-Benzyl-6,7,8,9-tetrahydro-5H-pyrazino [2,3-d] azepinhydrochlorid

10,9 g (30 mMol) 1-Benzyl-2,3,6,7-tetrahydro-4,5-bis(trimethylsiloxy)-azepin werden in 20 ml Äthylendiamin und 30 ml Tetraäthylenglykoldimethyläther gelöst. Bei 120 °C leitet man 8 Stunden lang Luft durch das Reaktionsgemisch. Dann gibt man auf Wasser und extrahiert mit Chloroform. Nach Einengen der getrockneten Chloroformextrakte wird an Kieselgel mit Methanol als Fließmittel chromatographisch gereinigt. Anschließend wird in Äthanol das Hydrochlorid gefällt.

Ausbeute : 2,4 g (29 % der Theorie),
Schmelzpunkt : 244-247 °C.

| | | | | |
|---|---|---|---|---|
| Ber. : | C 65,33 | H 6,58 | N 15,24 | Cl 12,85 |
| Gef. : | 65,01 | 6,42 | 14,89 | 12,31 |

b) 2-Amino-7-benzyl-6,7,8,9-tetrahydro-5H-pyrazino [2,3-d] azepin

2,4 g (0,01 Mol) 7-Benzyl-6,7,8,9-tetrahydro-5H-pyrazino [2,3-d] azepin werden in 20 ml Dimethylanilin mit 3,9 g (0,1 Mol) Natriumamid versetzt und 4 Stunden auf 170 °C erhitzt. Anschließend wird das Dimethylanilin im Hochvakuum abdestilliert. Der Rückstand wird in 4N-Natronlauge aufgenommen und mit Chloroform extrahiert. Die Extrakte werden getrocknet und an Kieselgel mit Methanol/Methylenchlorid = 3 : 1 als Fließmittel säulenchromatographisch gereinigt.

Ausbeute : 0,51 g (20 % der Theorie),
Schmelzpunkt : 110-112 °C.

| | | | |
|---|---|---|---|
| Ber. : | C 70,84 | H 7,13 | N 22,03 |
| Gef. : | 70,31 | 6,99 | 21,75 |

Beispiel 22

2-Amino-7-cyclopropyl-6,7,8,9-tetrahydro-5H-pyrazino [2,3-d] azepin-hydrochlorid

Zu 15,1 g (191,3 mMol) Pyridin in 200 ml absolutem Dioxan tropft man bei Raumtemperatur und unter Rühren 7,64 g (47,8 mMol) Brom. Die erhaltene Lösung tropft man unter leichter Kühlung zu 15 g (47,8 mMol) 1-Cyclopropyl-2,3,6,7-tetrahydro-4,5-bis(trimethylsilyloxy)-azepin in 200 ml absolutem Dioxan. Anschließend wird die entstandene Reaktionsmischung unter Eiskühlung rasch zu einer Lösung von 7,0 g (47,8 mMol) α-Amino-acetamidin-dihydrochlorid in 200 ml Wasser zugetropft. Nach 3 Stunden Nachrühren wird im Vakuum eingeengt, mit 2 N Natronlauge alkalisch gestellt und mit Chloroform extrahiert. Nach dem Trocknen der Extrakte über Magnesiumsulfat wird eingeengt und der kristalline Rückstand aus Acetonitril umkristallisiert. Die erhaltene kristalline Base wird in Äthanol gelöst und mit äthanolischer Salzsäure in das Hydrochlorid überführt.

Ausbeute : 7,45 g (67,7 % der Theorie),
Schmelzpunkt : 230 °C (Zers.).

| | C | H | N | Cl |
|---|---|---|---|---|
| Ber. : | 54,88 | 7,12 | 23,27 | 14,72 |
| Gef. : | 54,63 | 6,96 | 23,10 | 14,85 |

Beispiel 23

2-Amino-7-cyclobutyl-6,7,8,9-tetrahydro-5H-pyrazino [2,3-d] azepindihydrochlorid

Hergestellt analog Beispiel 22 aus α-Aminoacetamidin-dihydrochlorid und 1-Cyclobutyl-2,3,6,7-tetrahydro-4,5-bis-(trimethylsilyloxy)-azepin.

Ausbeute : 51 % der Theorie,
Schmelzpunkt : 265-270 °C (Zers.).

| | C | H | N | Cl |
|---|---|---|---|---|
| Ber. : | 49,49 | 6,92 | 19,24 | 24,35 |
| Gef. : | 49,95 | 7,15 | 19,18 | 24,40 |

Beispiel 24

2-Amino-6,7,8,9-tetrahydro-7-(2-Phenyl-äthyl)-5H-pyrazino [2,3-d] azepin-dihydrochlorid

Hergestellt analog Beispiel 22 aus 2,3,6,7-Tetrahydro-1-(2-phenyl-äthyl)-4,5-bis(trimethylsilyloxy)-azepin und α-Amino-acetamidin-dihydrochlorid.

Ausbeute : 53 % der Theorie,
Schmelzpunkt : 216-220 °C (Zers.).

| | C | H | N | Cl |
|---|---|---|---|---|
| Ber. : | 56,31 | 6,50 | 16,42 | 20,78 |
| Gef. : | 56,76 | 6,74 | 16,74 | 20,58 |

Beispiel 25

2-Amino-6,7,8,9-tetrahydro-7-(3-phenyl-propyl)-5H-pyrazino [2,3-d] azepin-dihydrochlorid

Hergestellt analog Beispiel 22 aus 2,3,6,7-Tetrahydro-1-(3-phenyl-propyl)-4,5-bis(trimethylsilyloxy)-azepin und α-Aminoacetamidin-dihydrochlorid.

Ausbeute : 39,5 % der Theorie,
Schmelzpunkt : 228-230 °C (Zers.)

| | C | H | N | Cl |
|---|---|---|---|---|
| Ber. : | 57,48 | 6,81 | 15,77 | 19,96 |
| Gef. : | 58,10 | 6,83 | 15,86 | 19,90 |

Beispiel 26

2-Amino-6,7,8,9-tetrahydro-5(R,S),7,9(R,S)-trimethyl-5H-pyrazino [2,3-d] azepin-dihydrochlorid

Hergestellt analog Beispiel 22 aus 2,3,6,7-Tetrahydro-1,3(R,S),6(R,S)-trimethyl-4,5-bis(trimethylsilyloxy)-azepin und α-Aminoacetamidin-dihydrochlorid.

Ausbeute : 20 % der Theorie,
Schmelzpunkt : 240-242 °C (Zers.)

| | C | H | N |
|---|---|---|---|
| Ber. : | 47,32 | 7,22 | 20,07 |
| Gef. : | 47,76 | 7,47 | 19,75 |

Beispiel 27

7-Äthyl-2-amino-6,7,8,9-tetrahydro-5(R,S)-methyl-5H-pyrazino [2,3-d] azepin-dihydrochlorid und 7-Äthyl-3-amino-6,7,8,9-tetrahydro-5(R,S)-methyl-5H-pyrazino [2,3-d] azepin-dihydrochlorid

Hergestellt analog Beispiel 22 aus 1-Äthyl-2,3,6,7-tetrahydro-3(R,S)-methyl-4,5-bis(trimethylsilyloxy)-azepin und α-Aminoacetamidin-dihydrochlorid.
Ausbeute : 17,5 % der Theorie,
Schmelzpunkt : 219-220 °C (Zers.)

| | | | |
|---|---|---|---|
| Ber. : | C 47,32 | H 7,22 | N 20,07 |
| Gef. : | 47,80 | 7,29 | 19,80 |

Beispiel 28

2-Amino-6,7,8,9-tetrahydro-5H-pyrazino [2,3-d] azepin-dihydrobromid

11,5 g (45 mMol) 2-Amino-7-benzyl-6,7,8,9-tetrahydro-5H-pyrazino [2,3-d] azepin und 11,6 g (90 mMol) Diisopropyläthylamin werden in 100 ml Methylenchlorid gelöst und mit 15,4 g (90 mMol) Chlorameisensäurebenzylester tropfenweise versetzt. Nach 48 Stunden Rühren wird im Vakuum eingeengt. Den Rückstand löst man in 50 ml Eisessig und 50 ml mit Bromwasserstoff gesättigtem Eisessig. Nach zweistündigem Erhitzen auf 100 °C engt man ein, verreibt mit Eisessig und saugt vom gebildeten Kristallbrei ab. Anschliessend wird mit Essigsäureäthylester nachgewaschen.
Ausbeute : 13,8 g (94 % der Theorie),
Schmelzpunkt : 240-243 °C

Beispiel 29

2-Amino-7-(4-chlor-phenylacetyl)-6,7,8,9-tetrahydro-5H-pyrazino [2,3-d] azepin

5,8 g (17,9 mMol) 2-Amino-6,7,8,9-tetrahydro-5H-pyrazino [2,3-d] azepin-dihydrobromid werden in 50 ml 2 N Natronlauge gelöst und unter Eiskühlung mit einer Lösung von 7,5 g 4-Chlorphenylacetylchlorid in 50 ml Dioxan tropfenweise versetzt. Nach Rühren über Nacht wird eingeengt und mit Chloroform extrahiert. Der Einengungsrückstand der getrockneten Chloroformextrakte wird mit Tetrachlorkohlenstoff verrieben und abgesaugt.
Ausbeute : 3,6 g (63 % der Theorie),
Schmelzpunkt : 183-187 °C

| | | | | |
|---|---|---|---|---|
| Ber. : | C 60,66 | H 5,41 | N 17,68 | Cl 11,19 |
| Gef. : | 60,29 | 5,20 | 17,54 | 11,01 |

Beispiel 30

2-Amino-7-(4-chlor-benzoyl)-6,7,8,9-tetrahydro-5H-pyrazino [2,3-d] azepin

Hergestellt analog Beispiel 29 aus 2-Amino-6,7,8,9-tetrahydro-5H-pyrazino [2,3-d] azepin-dihydrobromid und 4-Chlor-benzoylchlorid.
Ausbeute : 82,5 % der Theorie,
Schmelzpunkt : 189-191 °C

| | | | | |
|---|---|---|---|---|
| Ber. : | C 59,50 | H 4,99 | N 18,51 | Cl 11,71 |
| Gef. : | 59,53 | 4,71 | 18,41 | 11,93 |

Beispiel 31

2-Amino-7-(4-chlor-benzyl)-6,7,8,9-tetrahydro-5H-pyrazino [2,3-d] azepin

3,0 g (9,2 mMol) 2-Amino-6,7,8,9-tetrahydro-5H-pyrazino [2,3-d] azepin-dihydrobromid werden in 100 ml Acetonitril suspendiert und nach Zusatz von 7,5 ml Diisopropyläthylamin tropfenweise mit 1,5 g (9,3 mMol) 4-Chlor-benzylchlorid versetzt. Nach 48 Stunden Rühren wird eingeengt, mit verdünnter Salzsäure angesäuert und mit Methylenchlorid extrahiert. Anschließend wird die wässrige Phase alkalisch gestellt und mit Methylenchlorid erschöpfend extrahiert. Die eingeengten Extrakte werden über Kieselgel mit Essigsäureäthylester als Fließmittel säulenchromatographisch gereinigt.
Ausbeute : 480 mg (18,7 % der Theorie),
Schmelzpunkt : 158-160 °C

| | | | | |
|---|---|---|---|---|
| Ber. : | C 62,39 | H 5,93 | N 19,40 | Cl 12,28 |
| Gef. : | 62,70 | 6,10 | 19,33 | 12,37 |

Beispiel 32

2-Amino-7-[2-(4-chlor-phenyl)-äthyl]-6,7,8,9-tetrahydro-5H-pyrazino [2,3-d] azepin-dihydrochlorid

3 g (9,5 mMol) 2-Amino-7-(4-chlor-phenylacetyl)-6,7,8,9-tetrahydro-5H-pyrazino [2,3-d] azepin werden unter Rühren portionsweise zu einer Suspension von 3,8 g (0,1 Mol) Lithiumaluminiumhydrid in 150 ml absolutem Tetrahydrofuran gegeben. Nach Rühren über Nacht wird mit 2 N Natronlauge zersetzt, vom Natriumaluminat abgesaugt und eingeengt. Der Einengungsrückstand wird an Kieselgel im Fließmittel Essigsäureäthylester/Methanol (2 : 1) säulenchromatographisch gereinigt und anschließend mit äthanolischer Salzsäure das Dihydrochlorid gefällt.

Ausbeute : 0,5 g (14 % der Theorie),
Schmelzpunkt : 175-180 °C

| | | | | |
|---|---|---|---|---|
| Ber. : | C 51,15 | H 5,63 | N 14,91 | Cl 28,31 |
| Gef. : | 51,54 | 5,94 | 14,51 | 27,98 |

Beispiel 33

7-Äthyl-2-amino-6,7,8,9-tetrahydro-3-methyl-5H-pyrazino [2,3-d] azepin-dihydrochlorid

Hergestellt analog Beispiel 22 aus α-Amino-propionamidin-dihydrobromid und 1-Äthyl-2,3,6,7-tetrahydro-4,5-bis(trimethylsilyloxy)-azepin.

Ausbeute : 48 % der Theorie,
Schmelzpunkt : 263 °C (Zers.)

| | | | | |
|---|---|---|---|---|
| Ber. : | C 47,32 | H 7,22 | N 20,07 | Cl 25,40 |
| Gef. : | 47,19 | 7,48 | 19,81 | 24,90 |

Beispiel 34

3,7-Diäthyl-2-amino-6,7,8,9-tetrahydro-5H-pyrazino [2,3-d] azepin-dihydrochlorid

Hergestellt analog Beispiel 22 aus α-Amino-butyramidin-dihydrobromid und 1-Äthyl-2,3,6,7-tetrahydro-4,5-bis(trimethylsilyloxy)-azepin.

Ausbeute : 46,5 % der Theorie,
Schmelzpunkt : 247 °C (Zers.)

| | | | | |
|---|---|---|---|---|
| Ber. : | C 49,15 | H 7,56 | N 19,11 | Cl 24,18 |
| Gef. : | 49,35 | 7,64 | 18,90 | 24,38 |

Beispiel 35

7-Äthyl-2-amino-6,7,8,9-tetrahydro-3-phenyl-5H-pyrazino [2,3-d] azepin-dihydrochlorid

Hergestellt analog Beispiel 22 aus α-Phenyl-α-amino-acetamidin-dihydrobromid und 1-Äthyl-2,3,6,7-tetrahydro-4,5-bis-(trimethylsilyloxy)-azepin.

Ausbeute : 54 % der Theorie,
Schmelzpunkt : 245-247 °C (Zers.)

| | | | | |
|---|---|---|---|---|
| Ber. : | C 56,31 | H 6,50 | N 16,42 | Cl 20,78 |
| Gef. : | 56,30 | 6,71 | 16,26 | 20,42 |

Beispiel 36

7-Äthyl-2-amino-6,7,8,9-tetrahydro-5H-pyrazino [2,3-d] azepin-3-carbonsäure-äthylester-dihydrochlorid

Hergestellt analog Beispiel 22 aus 1-Äthyl-2,3,6,7-tetrahydro-4,5-bis(trimethylsilyloxy)-azepin und 2-Amidino-2-amino-essigsäureäthylester-dihydrochlorid.

Ausbeute : 17,5 % der Theorie,
Schmelzpunkt : 217 °C

| | | | | |
|---|---|---|---|---|
| Ber. : | C 46,30 | H 6,58 | N 16,61 | Cl 21,03 |
| Gef. : | 46,35 | 6,65 | 16,85 | 21,05 |

Beispiel 37

7-Äthyl-2-amino-3-chlor-6,7,8,9-tetrahydro-5H-pyrazino [2,3-d]azepin-hydrochlorid

6 g (31,3 mMol) 7-Äthyl-2-amino-6,7,8,9-tetrahydro-5H-pyrazino [2,3-d] azepin werden in 100 ml Eisessig gelöst. Unter Eiskühlung leitet man Chlor ein bis im Dünnschichtchromatogramm kein Ausgangsmaterial mehr nachzuweisen ist. Anschließend stellt man mit 2N Natronlauge alkalisch und extrahiert mit Chloroform. Die Chloroformextrakte werden eingeengt und an Kieselgel im Fließmittel Essigsäureäthylester/Methanol/äthanolischem Ammoniak (4 : 1 : 0,5) säulenchromatographisch gereinigt. Dann wird an Äthanol das Hydrochlorid gefällt.

Ausbeute : 1,8 g (21,8 % der Theorie),
Schmelzpunkt : 306 °C (Zers.)

| | | | | |
|---|---|---|---|---|
| Ber. : | C 45,64 | H 6,13 | N 21,29 | Cl 26,94 |
| Gef. : | 45,80 | 6,11 | 21,22 | 27,10 |

Beispiel 38

7-Äthyl-2-amino-3-brom-6,7,8,9-tetrahydro-5H-pyrazino [2,3-d] azepin-hydrochlorid

Hergestellt analog Beispiel 37 aus 7-Äthyl-2-amino-6,7,8,9-tetrahydro-5H-pyrazino [2,3-d] azepin und Brom.
Ausbeute : 64,5 % der Theorie,
Schmelzpunkt : 312 °C (Zers.)

| | | | | | |
|---|---|---|---|---|---|
| Ber. : | C 39,04 | H 5,24 | N 18,21 | Cl 11,52 | Br 25,97 |
| Gef. : | 38,81 | 5,06 | 18,65 | 11,65 | 26,20 |

Beispiel 39

7-Äthyl-2-amino-6,7,8,9-tetrahydro-3-methoxy-5H-pyrazino [2,3-d] azepin-dihydrochlorid

1 g (3,3 mMol) 7-Äthyl-2-amino-3-brom-6,7,8,9-tetrahydro-5H-pyrazino [2,3-d] azepin-hydrochlorid werden mit einer Lösung von 1 g (43,5 mMol) Natrium in 50 ml Methanol versetzt und 10 Stunden im Autoklaven bei 150 °C gerührt. Anschließend wird eingeengt, mit 2 N Natronlauge aufgenommen und mit Chloroform extrahiert. Die Extrakte werden eingeengt und der Rückstand in Aceton mit äthanolischer Salzsäure versetzt. Man erhält das Dihydrochlorid in Form weißer Kristalle.
Ausbeute : 150 mg (15,4 % der Theorie),
Schmelzpunkt : ab 150 °C, klar bei 245 °C.

| | | | | |
|---|---|---|---|---|
| Ber. : | C 44,75 | H 6,83 | N 18,98 | Cl 24,02 |
| Gef. : | 44,65 | 6,85 | 19,00 | 24,05 |

Beispiel 40

2-Amino-7-benzyl-6,7,8,9-tetrahydro-5H-pyrazino [2,3-] azepin

24 g (66 mMol) 1-Benzyl-5-brom-hexahydro-azepin-4-on-hydrobromid (hergestellt durch Bromieren von 1-Benzyl-hexahydro-azepin-4-on mit Brom in Eisessig) werden in Äthanol gelöst und nach Zugabe von 4,6 g (66 mMol) Hydroxylaminhydrochlorid, tropfenweise mit einer Lösung von 1,5 g (65,2 mMol) Natrium in 20 ml Äthanol versetzt. Nach Rühren über Nacht bei 30-40 °C wird filtriert und das Filtrat eingeengt. Zum Einengungsrückstand werden anschließend 6,1 g (66 mMol) α-Amino-acetonitril-hydrochlorid und 100 ml Chloroform gegeben. Unter Rühren tropft man 27,7 ml (0,2 Mol) Triäthylamin zu und läßt dann über Nacht nachrühren. Nach dem Einengen wird mit 2 N Natronlauge versetzt und mit Chloroform extrahiert. Die getrockneten und eingeengten Extrakte werden an Kieselgel im Fließmittel Toluol/Essigsäureäthylester (2 : 1) säulenchromatographisch gereinigt. Zuletzt wird aus Tetrachlorkohlenstoff umkristallisiert.
Ausbeute : 2,5 g (15 % der Theorie),
Schmelzpunkt : 115-118 °C.

| | | | |
|---|---|---|---|
| Ber. : | C 70,84 | H 7,13 | N 22,03 |
| Gef. : | 70,64 | 7,13 | 22,03 |

Beispiel 41

7-Äthyl-2-amino-6,7,8,9-tetrahydro-5H-pyrazino [2,3-d] azepin

Hergestellt aus 1-Äthyl-5-brom-hexahydro-azepin-4-on-hydrobromid, Hydroxylamin und α-Aminoacetonitril-hydrochlorid analog Beispiel 40.
Ausbeute : 6 % der Theorie,
Schmelzpunkt : 83-84 °C.

| | | | |
|---|---|---|---|
| Ber. : | C 62,47 | H 8,39 | N 29,14 |
| Gef. : | 62,31 | 8,07 | 28,95 |

Beispiel 42

7-Äthyl-2-amino-6,7,8,9-tetrahydro-6(R,S)-methyl-5H-pyrazino [2,3-d] azepin-dihydrochlorid und 7-Äthyl-3-amino-6,7,8,9-tetrahydro-6(R,S)-methyl-5H-pyrazino [2,3-d] azepin-dihydrochlorid

Hergestellt aus 1-Äthyl-2,3,6,7-tetrahydro-2(R,S)-methyl-4,5-bis(trimethylsilyloxy)-azepin und α-Amino-acetamidin-dihydrochlorid analog Beispiel 22.
Ausbeute : 11 % der Theorie,
Schmelzpunkt : < 200 °C.
Ber. : C 47,32 H 7,22 N 20,06
Gef. : 47,21 7,01 19,56

Beispiel 43

7-Äthyl-2-amino-6,7,8,9-tetrahydro-5H-pyrazino [2,3-d] azepin

Hergestellt durch Alkylierung von 2-Amino-6,7,8,9-tetrahydro-5H-pyrazino [2,3-] azepin-dihydrobromid analog Beispiel 31.
Ausbeute : 34 % der Theorie,
Schmelzpunkt : 84-85 °C.
Ber. : Molpeak m/e = 192
Gef. : Molpeak m/e = 192

Beispiel 44

6-Äthyl-2-amino-5,6,7,8-tetrahydro-pyrido [3,4-b] pyrazin-dihydrochlorid

Hergestellt aus 1-Äthyl-1,2,5,6-tetrahydro-3,4-bis(trimethylsilyloxy)-pyridin und α-Amino-acetamidin-dihydrochlorid analog Beispiel 22.
Ausbeute : 14 % der Theorie,
Schmelzpunkt : 255-258 °C.
Ber. : C 43,04 H 6,42 N 22,31 Cl 28,23
Gef. : 43,35 6,64 22,53 27,80

Beispiel 45

6-Äthyl-3-amino-5,6,7,8-tetrahydro-pyrido [3,4-b] pyrazin-dihydrochlorid

a) 6-Acetyl-3-amino-5,6,7,8-tetrahydro-pyrido [3,4-b] pyrazin-4-oxid

Hergestellt aus 1-Acetyl-piperidin-4-on und tert. Butylnitrit analog Beispiel A.
Schmelzpunkt ö 235-237 °C (Zers.).

b) 6-Acetyl-3-amino-5,6,7,8-tetrahydro-pyrido [3,4-b] pyrazin

Hergestellt aus 6-Acetyl-3-amino-5,6,7,8-tetrahydro-pyrido [3,4-b] pyrazin-4-oxid durch Reduktion mit Natriumdithionit analog Beispiel 19.
Ausbeute : 46,9 % der Theorie,
Schmelzpunkt : 183-185 °C.
Ber. : C 56,24 H 6,29 N 29,15
Gef. : 56,12 6,14 28,52

c) 6-Äthyl-3-amino-5,6,7,8-tetrahydro-pyrido [3,4-b] pyrazin-dihydrochlorid

Hergestellt aus 6-Acetyl-3-amino-5,6,7,8-tetrahydro-pyrido [3,4-b] pyrazin durch Lithiumaluminiumhydridreduktion analog Beispiel 17.
Ausbeute : 14,3 % der Theorie,
Schmelzpunkt : 249-251 °C (Zers.).
Ber. : C 43,04 H 6,42 N 22,31 Cl 28,23
Gef. : 43,41 6,57 22,10 28,02

Beispiel 46

2-amino-5,6,7,8-tetrahydro-6-methyl-pyrido [3,4-b] pyrazin-dihydrochlorid und 3-Amino-5,6,7,8-tetrahydro-6-methyl-pyrido [3,4-b] pyrazin-dihydrochlorid

Hergestellt aus 1,2,5,6-Tetrahydro-1-methyl-3,4-bis(trimethylsilyloxy)-pyridin und α-Amino-acetamidin-dihydrochlorid analog Beispiel 22.
Ausbeute : 9,1 % der Theorie,
Schmelzpunkt : 220-225 °C.

Ber.: C 40,51 H 5,95 N 23,62
Gef.: 41,25 6,25 23,60

Beispiel 47

2-Amino-5,6,7,8-tetrahydro-6-propyl-pyrido [3,4-b] pyrazin-dihydrochlorid und 3-Amino-5,6,7,8-tetrahydro-6-propyl-pyrido [3,4-b] pyrazin-dihydrochlorid

Hergestellt aus 1,2,5,6-Tetrahydro-1-propyl-3,4-bis(trimethylsilyloxy)-pyridin und α-Amino-acetamidin-dihydrochlorid analog Beispiel 22.
Ausbeute: 8,5 % der Theorie,
Schmelzpunkt: 222 °C.
Ber.: C 45,29 H 6,84 N 21,13
Gef.: 45,80 6,49 20,90

Beispiel 48

2-Amino-5,6,7,8-tetrahydro-6-isopropyl-pyrido [3,4-b] pyrazindihydrochlorid und 3-Amino-5,6,7,8-tetrahydro-6-isopropyl-pyrido [3,4-b] pyrazin-dihydrochlorid

Hergestellt aus 1,2,5,6-Tetrahydro-1-isopropyl-3,4-bis(trimethylsilyloxy)-pyridin und α-Amino-acetamidin-dihydrochlorid analog Beispiel 22.
Ausbeute: 10,2 % der Theorie,
Schmelzpunkt: 215-220 °C.
Ber.: C 45,29 H 6,84 N 21,13 Cl 26,74
Gef.: 45,27 6,91 21,22 26,35

Beispiel 49

2-Amino-6-benzyl-5,6,7,8-tetrahydro-pyrido [3,4-b] pyrazin-dihydrochlorid und 3-Amino-6-benzyl-5,6,7,8-tetrahydro-pyrido [3,4-b] pyrazin-dihydrochlorid

Hergestellt aus 1-Benzyl-1,2,5,6-tetrahydro-3,4-bis(trimethylsilyloxy)-pyridin und α-Amino-acetamidin-dihydrochlorid analog Beispiel 22.
Ausbeute: 19 % der Theorie,
Schmelzpunkt: 261 °C (Zers.)
Ber.: C 53,68 H 5,79 N 17,89 Cl 22,64
Gef.: 54,05 5,76 18,07 22,40

Beispiel 50

2-Amino-6,7,8,9-tetrahydro-7-isopropyl-5H-pyrazino [2,3-d] azepin-dihydrochlorid

1,64 g (10 mMol) 2-Amino-6,7,8,9-tetrahydro-5H-pyrazino [2,3-d] azepin werden in 100 ml Isopropanol gelöst und nach Zugabe von 16 g Raney-Nickel 6 Stunden am Rückfluß gekocht. Anschließend wird vom Raney-Nickel abfiltriert, das Filtrat eingeengt und zur Reinigung an Kieselgel chromatographiert (Fließmittel: Methanol/Methylenchlorid = 3 : 1). Anschließend wird in Äthanol mit äthanolischer Salzsäure das Dihydrochlorid gefällt und abgesaugt.
Ausbeute: 1,5 g (53,7 % der Theorie),
Schmelzpunkt: 221-223 °C.

Beispiel 51

6-Äthyl-2-amino-6,7-dihydro-5H-pyrrolo [3,4-b] pyrazin

Hergestellt aus 1-Äthyl-2,5-dihydro-3,4-bis(trimethylsilyloxy)-pyrrol und α-Amino-acetamidin-dihydrochlorid analog Beispiel 22.
Ausbeute: 7 % der Theorie,
Schmelzpunkt: < 20 °C.
Ber.: Molpeak m/e = 164
Gef.: Molpeak m/e = 164.

Beispiel I

Dragéekern mit 5 mg 7-Äthyl-2-amino-6,7,8,9-tetrahydro-5H-pyrazino [2,3-d] azepin-hydrochlorid

Zusammensetzung :

1 Dragéekern enthält :

| | |
|---|---|
| 7-Äthyl-2-amino-6,7,8,9-tetrahydro-5H-pyrazino [2,3-d] azepin-hydrochlorid | 5,0 mg |
| Milchzucker | 33,5 mg |
| Maisstärke | 10,0 mg |
| Gelatine | 1,0 mg |
| Magnesiumstearat | 0,5 mg |
| | 50,0 mg |

Herstellungsverfahren :

Die Mischung der Wirksubstanz mit Milchzucker und Maisstärke wird mit einer 10 %igen wäßrigen Gelatinelösung durch Sieb 1 mm granuliert, bei 40 °C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

Kerngewicht : 50 mg
Stempel : 5 mm, gewölbt

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht : 100 mg

Beispiel II

Tropfen mit 5 mg 7-Äthyl-2-amino-6,7,8,9-tetrahydro-5H-pyrazino [2,3-d] azepin-dihydrochlorid pro 1 ml

Zusammensetzung :
100 ml Tropflösung enthalten :

| | |
|---|---|
| p-Oxybenzoesäuremethylester | 0,035 g |
| p-Oxybenzoesäurepropylester | 0,015 g |
| Anisöl | 0,05 g |
| Menthol | 0,06 g |
| Äthanol rein | 10,0 g |
| 7-Äthyl-3-amino-6,7,8,9-tetrahydro-5H-pyrazino [2,3-d] azepin-dihydrochlorid | 0,5 g |
| Zitronensäure | 0,7 g |
| Natriumphosphat sek. × 2 $H_2O$ | 0,3 g |
| Natriumcyclamat | 1,0 g |
| Glycerin | 15,0 g |
| Dest. Wasser ad | 100,0 ml |

Herstellungsverfahren :

Die p-Oxybenzoesäureester, Anisöl sowie Menthol werden in Äthanol gelöst (Lösung I).

Die Puffersubstanzen, die Wirksubstanz und Natriumcyclamat werden in dest. Wasser gelöst und Glycerin zugefügt (Lösung II). Lösung I wird in Lösung II eingerührt und die Mischung mit dest. Wasser auf das gegebene Volumen aufgefüllt. Die fertige Tropflösung wird durch ein geeignetes Filter filtriert. Die Herstellung und Abfüllung der Tropflösung muß unter Lichtschutz und unter Schutzbegasung erfolgen.

Herstellungsverfahren :

In ausgekochtem und unter $CO_2$-Begasung abgekühltem Wasser werden nacheinander die Puffersubstanzen, die Wirksubstanz Natriumpyrosulfit gelöst. Man füllt mit abgekochtem Wasser auf das gegebene Volumen auf und filtriert pyrogenfrei.

Abfüllung : in braune Ampullen unter Schutzbegasung
Sterilisation : 20 Minuten bei 120 °C.

Die Herstellung und Abfüllung der Ampullenlösung muß in abgedunkelten Räumen vorgenommen werden.

Beispiel III

Suppositorien mit 10 mg 2-Amino-6,7,8,9-tetrahydro-7-methyl-5H-pyrazino [2,3-d] azepin-dihydrochlorid

1 Zäpfchen enthält :

| | |
|---|---|
| 2-Amino-6,7,8,9-tetrahydro-7-methyl-5H-pyrazino [2,3-d] azepin-dihydrochlorid | 10,0 mg |
| Zäpfchenmasse (z. B. Witepsol W 45) | 1 690,0 mg |
| | 1 700,0 mg |

Herstellungsverfahren :

Die feingepulverte Substanz wird mit Hilfe eines Eintauchhomogenisators in die geschmolzene und auf 40 °C abgekühlte Zäpfchenmasse eingerührt. Die Masse wird bei 35 °C in leicht vorgekühlte Formen ausgegossen.

Zäpfchengewicht : 1,7 g

Beispiel IV

Ampullen mit 5 mg 7-Allyl-2-amino-6,7,8,9-tetrahydro-5H-pyrazino [2,3-d] azepin-dihydrochlorid

1 Ampulle enthält :

| | |
|---|---|
| 7-Allyl-2-amino-6,7,8,9-tetrahydro-5H-pyrazino [2,3-d] azepin-dihydrochlorid | 5,0 mg |
| Zitronensäure | 7,0 mg |
| Natriumphosphat sek. × 2 $H_2O$ | 3,0 mg |
| Natriumpyrosulfit | 1,0 mg |
| Dest. Wasser ad | 1,0 ml |

Beispiel V

Dragées mit 1 mg 7-Äthyl-2-amino-6,7,8,9-tetrahydro-5H-pyrazino [2,3-d] azepin-dihydrochlorid

1 Dragéekern enthält :

| | |
|---|---|
| 7-Äthyl-2-amino-6,7,8,9-tetrahydro-5H-pyrazino [2,3-d] azepin-dihydrochlorid | 1,0 mg |
| Milchzucker | 35,5 mg |
| Maisstärke | 12,0 mg |
| Gelatine | 1,0 mg |
| Magnesiumstearat | 0,5 mg |
| | 50,0 mg |

Herstellungsverfahren :

Analog Beispiel I.

| | |
|---|---|
| Kerngewicht : | 50 mg |
| Stempel : | 5 mm, gewölbt |
| Dragéegewicht : | 100 mg |

**Ansprüche** (für die Vertragsstaaten : BE, CH, DE, FR, IT, LI, LU, NL, SE)

1. Neue Pyrazine der allgemeinen Formel

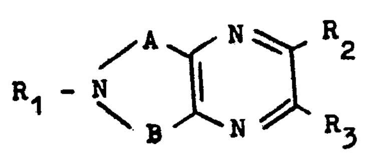

(I)

in der

A und B, die gleich oder verschieden sein können, jeweils eine gegebenenfalls durch eine Alkylgruppe mit 1 bis 3 Kohlenstoffatomen substituierte Methylen- oder Äthylengruppe,

$R_1$ ein Wasserstoffatom, eine Alkylgruppe mit 4 bis 6 Kohlenstoffatomen, eine Cycloalkylgruppe mit 3 bis 7 Kohlenstoffatomen, eine Alkenylgruppe mit 3 bis 6 Kohlenstoffatomen, eine Alkyl- oder Alkanoylgruppe mit jeweils 1 bis 3 Kohlenstoffatomen, welche jeweils durch eine gegebenenfalls durch ein Halogenatom substituierte Phenylgruppe substituiert sein kann, eine Alkoxycarbonylgruppe mit

insgesamt 2 bis 4 Kohlenstoffatomen, eine Aralkoxycarbonylgruppe mit insgesamt 8 bis 10 Kohlenstoffatomen oder die Phenylgruppe,

einer der Reste $R_2$ oder $R_3$ eine Aminogruppe und

der andere der Reste $R_2$ oder $R_3$ ein Wasserstoff-, Chlor- oder Bromatom, eine Alkylgruppe mit 1 bis 3 Kohlenstoffatomen, eine Alkoxygruppe mit 1 bis 3 Kohlenstoffatomen, eine Alkoxycarbonylgruppe mit insgesamt 2 bis 4 Kohlenstoffatomen, eine Phenyl- oder Halogenphenylgruppe bedeuten, und deren Säureadditionssalze, insbesondere deren physiologisch verträgliche Salze mit anorganischen oder organischen Säuren.

2. Neue Verbindungen der allgemeinen Formel I gemäß Anspruch 1, in der

A und B, die gleich oder verschieden sein können, jeweils eine Methylen-, Äthylen- oder Methyläthylengruppe,

$R_1$ ein Wasserstoffatom, eine Alkylgruppe mit 1 bis 4 Kohlenstoffatomen, eine Cycloalkylgruppe mit 3 bis 6 Kohlenstoffatomen, eine Alkenylgruppe mit 3 oder 4 Kohlenstoffatomen, eine durch eine Phenyl- oder Chlorphenylgruppe substituierte Alkylgruppe mit 1 bis 3 Kohlenstoffatomen, eine gegebenenfalls durch eine Phenyl- oder Chlorphenylgruppe substituierte Formyl- oder Acetylgruppe, eine Äthoxycarbonyl- oder Phenylgruppe,

$R_2$ ein Wasserstoff-, Chlor- oder Bromatom, eine Methyl-, Äthyl-, Methoxy-, Phenyl- oder Äthoxycarbonylgruppe und

$R_3$ eine Aminogruppe bedeuten, und deren Säureadditionssalze, insbesondere deren physiologisch verträgliche Salze mit anorganischen oder organischen Säuren.

3. Neue Verbindungen der allgemeinen Formel I gemäß Anspruch 1, in der

A ein Methylen- oder Äthylengruppe,

B die Äthylengruppe,

$R_1$ eine Methyl-, Äthyl-, Isopropyl-, Cyclopropyl-, Cyclobutyl- oder Allylgruppe,

$R_2$ eine Methylgruppe, ein Wasserstoff-, Chlor- oder Bromatom und

$R_3$ eine Aminogruppe bedeuten, und deren physiologisch verträgliche Säureadditionssalze mit anorganischen oder organischen Säuren.

4. Neue Verbindungen der allgemeinen Formel I gemäß Anspruch 1, in der

B, $R_1$ und $R_3$ wie im Anspruch 3 definiert sind,

A die Äthylengruppe und

$R_2$ ein Wasserstoffatom bedeuten, und deren physiologisch verträgliche Säureadditionssalze mit anorganischen oder organischen Säuren.

5. 7-Äthyl-2-amino-6,7,8,9-tetrahydro-5H-pyrazino [2,3-d] azepin und dessen Säureadditionssalze.

6. 2-Amino-6,7,8,9-tetrahydro-7-methyl-5H-pyrazino [2,3-d] azepin und dessen Säureadditionssalze.

7. 6-Äthyl-2-amino-5,6,7,8-tetrahydro-pyrido [3,4-b] pyrazin und dessen Säureadditionssalze.

8. Arzneimittel, enthaltend eine Verbindung gemäß den Ansprüchen 1 bis 7 neben einem oder mehreren inerten Trägerstoffen oder Verdünnungsmitteln.

9. Verwendung einer Verbindung gemäß den Ansprüchen 1 bis 7 sur Herstellung eines Arzneimittels auf nichtchemischem Wege zur Behandlung von Herz- und Kreislauferkrankungen.

10. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe und der andere der Reste $R_2$ oder $R_3$ eine Aminogruppe darstellen, ein gegebenenfalls im Reaktionsgemisch gebildetes Diketon der allgemeinen Formel

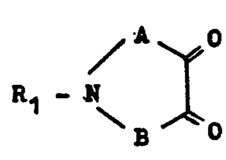

(II)

in der

A, B und $R_1$ wie in Anspruch 1 definiert sind, mit einem 2-Aminoacetamidin der allgemeinen Formel

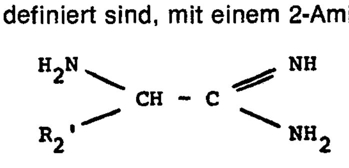

(III)

in der

$R_2'$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe bedeutet, in einem Lösungsmittel umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls surch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

11. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß ein gegebenenfalls im Reaktionsgemisch gebildetes Pyrazin-N-oxid der allgemeinen Formel

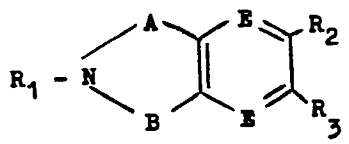

(IV)

in der

$R_1$ bis $R_3$, A und B wie in Anspruch 1 definiert sind, einer der Reste E eine N-Oxidgruppe und der andere der Reste E ein Stickstoffatom oder eine N-Oxidgruppe bedeuten, in einem Lösungsmittel reduziert wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

12. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Rest $R_2$ oder $R_3$ Wasserstoffatom oder eine Alkoxycarbonylgruppe mit insgesamt 2 bis 4 Kohlenstoffatomen bedeuten, eine gegebenenfalls im Reaktionsgemisch gebildete Verbindung der allgemeinen Formel

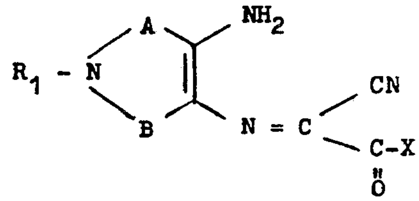

(V)

in der

$R_1$, A und B wie in Anspruch 1 definiert sind und

X eine Alkoxygruppe darstellt, in einem Lösungsmittel cyclisiert und gewünschtenfalls eine so erhaltene Verbindung der allgemeinen Formel

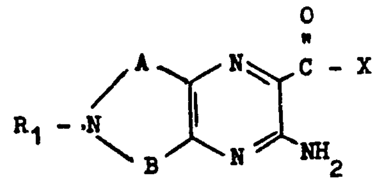

(VI)

in der

$R_1$, A, B und X wie oben definiert sind, hydrolysiert und decarboxyliert wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen,

eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

13. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Reste $R_2$ oder $R_3$ ein Wasserstoffatom darstellen, ein Pyrazin der allgemeinen Formel

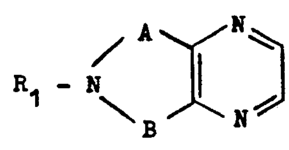

(VII)

in der

$R_1$, A und B wie in Anspruch 1 definiert sind, in einem Lösungsmittel mit einem Alkaliamid umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen,

eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit

insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

14. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Rest $R_2$ oder $R_3$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe darstellt, ein gegebenenfalls im Reaktionsgemisch gebildetes Oxim der allgemeinen Formel

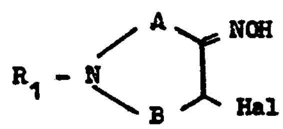
(VIII)

in der

$R_1$, A und B wie in Anspruch 1 definiert sind und

Hal ein Chlor-, Brom- oder Jodatom darstellt, mit einem 2-Amino-acetonitril der allgemeinen Formel

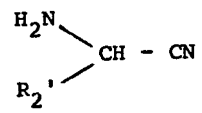
(IX)

in der

$R_2'$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe darstellt, in einem Lösungsmittel umgesetzt wird und

gewünschtenfalls anschließend eine erfindungsgemäß erhaltene Verbindung der allgemeinen Formel I, in der $R_1$ eine gegebenenfall durch eine Phenyl- oder Halogenphenylgruppe substituierte Alkanoylgruppe mit 1 bis 3 Kohlenstoffatomen,

eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

15. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der $R_1$ mit Ausnahme des Wasserstoffatoms und der Phenylgruppe die für $R_1$ in Anspruch 1 erwähnten Bedeutungen besitzt, ein Pyrazin der allgemeinen Formel

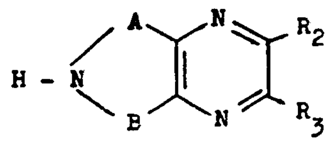

(XI)

in der

$R_2$, $R_3$, A und B wie in Anspruch 1 definiert sind, mit einer Verbindung der allgemeinen Formel

$$R_1'—Y \qquad (XII)$$

in der

$R_1'$ mit Ausnahme des Wasserstoffatoms und der Phenylgruppe die für $R_1$ in Anspruch 1 erwähnten Bedeutungen besitzt und

Y eine nukleophile Austrittsgruppe darstellt, in einem Lösungsmittel umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

16. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Reste $R_2$ oder $R_3$ eine Alkoxygruppe mit 1 bis 3 Kohlenstoffatomen darstellen, ein Pyrazin der allgemeinen Formel

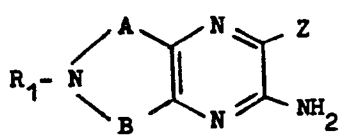

(XIII)

in der

$R_1$, A und B wie in Anspruch 1 definiert sind und

Z eine nukleophile Austrittsgruppe wie ein Halogenatom darstellt, mit einer Verbindung der allgemeinen Formel

$$H—R_2'' \qquad (XIV)$$

in der

$R_2''$ eine Alkoxygruppe mit 1 bis 3 Kohlenstoffatomen darstellt, oder dessen Alkalisalz in einem Lösungsmittel umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine

Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

**Ansprüche** (für den Vertragsstaat AT)

1. Verfahren zur Herstellung von neuen Pyrazinen der allgemeinen Formel

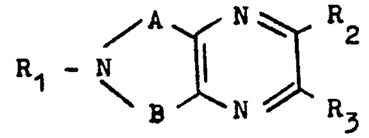

(I)

in der

A und B, die gleich oder verschieden sein können, jeweils eine gegebenenfalls durch eine Alkylgruppe mit 1 bis 3 Kohlenstoffatomen substituierte Methylen- oder Äthylengruppe,

$R_1$ ein Wasserstoffatom, eine Alkylgruppe mit 4 bis 6 Kohlenstoffatomen, eine Cycloalkylgruppe mit 3 bis 7 Kohlenstoffatomen, eine Alkenylgruppe mit 3 bis 6 Kohlenstoffatomen, eine Alkyl- oder Alkanoylgruppe mit jeweils 1 bis 3 Kohlenstoffatomen, welche jeweils durch eine gegebenenfalls durch ein Halogenatom substituierte Phenylgruppe substituiert sein kann, eine Alkoxycarbonylgruppe mit insgesamt 2 bis 4 Kohlenstoffatomen, eine Aralkoxycarbonylgruppe mit insgesamt 8 bis 10 Kohlenstoffatomen oder die Phenylgruppe,

einer der Reste $R_2$ oder $R_3$ eine Aminogruppe und

der andere der Reste $R_2$ oder $R_3$ ein Wasserstoff-, Chlor- oder Bromatom, eine Alkylgruppe mit 1 bis 3 Kohlenstoffatomen, eine Alkoxygruppe mit 1 bis 3 Kohlenstoffatomen, eine Alkoxycarbonylgruppe mit insgesamt 2 bis 4 Kohlenstoffatomen, eine Phenyl- oder Halogenphenylgruppe bedeugen, und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe und

der andere der Reste $R_2$ oder $R_3$ eine Aminogruppe darstellen, ein gegebenenfalls im Reaktionsgemisch gebildetes Diketon der allgemeinen Formel

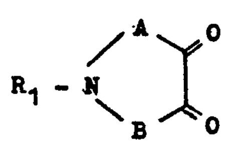

(II)

in der

A, B und $R_1$ wie eingangs definiert sind, mit einem 2-Amino-acetamidin der allgemeinen Formel

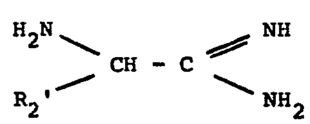

(III)

in der

$R_2'$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe bedeutet, in einem Lösungsmittel umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende

Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenation substituierte Benzylgruppe darstellt, durch Umsetzung mit Chlorameisensäurebenzylester und anschließend mit eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

2. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß ein gegebenenfalls im Reaktionsgemisch gebildetes Pyrazin-N-oxid der allgemeinen Formel

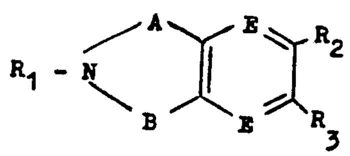

(IV)

in der

$R_1$ bis $R_3$, A und B wie im Anspruch 1 definiert sind, einer der Reste E eine N-Oxidgruppe und der andere der Reste E ein Stickstoffatom oder eine N-Oxidgruppe bedeuten, in einem Lösungsmittel reduziert wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

3. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Reste $R_2$ oder $R_3$ ein Wasserstoffatom oder eine Alkoxycarbonylgruppe mit insgesamt 2 bis 4 Kohlenstoffatomen beuden, eine gegebenenfalls im Reaktionsgemisch gebildete Verbindung der allgemeinen Formel

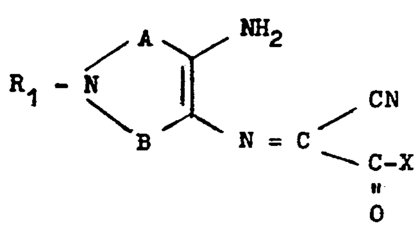

(V)

in der

$R_1$, A und B wie im Anspruch 1 definiert sind und

X eine Alkoxygruppe darstellt, in einem Lösungsmittel cyclisiert und gewünschtenfalls eine so erhaltene Verbindung der allgemeinen Formel

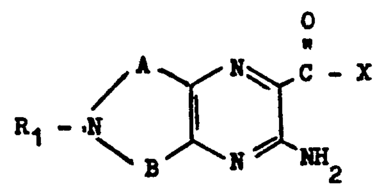

(VI)

in der

$R_1$, A, B und X wie oben definiert sind, hydrolysiert und decarboxyliert wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

4. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Reste $R_2$ oder $R_3$ ein Wasserstoffatom darstellen, ein Pyrazin der allgemeinen Formel

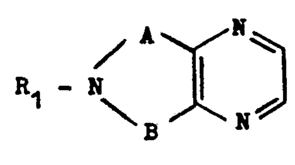

(VII)

in der

$R_1$, A und B wie im Anspruch 1 definiert sind, in einem Lösungsmittel mit einem Alkaliamid umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit Eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

5. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Reste $R_2$ oder $R_3$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe darstellt, ein gegebenenfalls im Reaktionsgemisch gebildetes Oxim der allgemeinen Formel

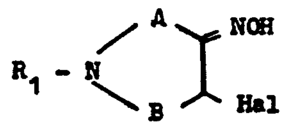

(VIII)

in der

$R_1$, A und B wie im Anspruch 1 definiert sind und

Hal ein Chlor-, Brom- oder Jodatom darstellt, mit einem 2-Amino-Acetonitril der allgemeinen Formel

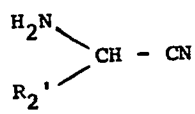

(IX)

in der

$R_2'$ ein Wasserstoffatom, eine Alkylgruppe mit 1 bis 3 Kohlenstoffatomen, eine Alkoxycarbonylgruppe mit insgesamt 2 bis 4 Kohlenstoffatomen, eine Phenyl- oder Halogenphenylgruppe darstellt, in einem Lösungsmittel umgesetzt wird und

gewunschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit eisessig/Bromwasserstoff in eine enstprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

6. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der $R_1$ mit Ausnahme des Wasserstoffatoms und der Phenylgruppe die für $R_1$ im Anspruch 1 erwähnten Bedeutungen besitzt, ein Pyrazin der allgemeinen Formel

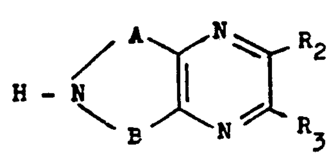

(XI)

in der

$R_2$, $R_3$, A und B wie im Anspruch 1 definiert sind, mit einer Verbindung der allgemeinen Formel

$$R_1'—Y \quad (XII)$$

in der

$R_1'$ mit Ausnahme des Wasserstoffatoms und der Phenylgruppe die für $R_1$ im Anspruch 1 erwähnten Bedeutungen besitzt und

Y eine nukleophile Austrittsgruppe darstellt, in einem Lösungsmittel umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein Halogenatom substituierte Benzylgruppe darstellt, durch Umsetzung mit Chlorameisensäurebenzylester und anschließend mit eisessig/Bromwasserstoff in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure über geführt wird.

7. Verfahren zur Herstellung von neuen Pyrazinen der allgemeinen Formel I gemäß Anspruch 1 und von deren Säureadditionssalzen, insbesondere von deren physiologisch verträglichen Salzen mit anorganischen oder organischen Säuren, dadurch gekennzeichnet, daß zur Herstellung von Verbindungen der allgemeinen Formel I, in der einer der Reste $R_2$ oder $R_3$ eine Aminogruppe und der andere der Reste $R_2$ oder $R_3$ eine Alkoxygruppe mit 1 bis 3 Kohlenstoffatomen darstellen, ein Pyrazin der allgemeinen Formel

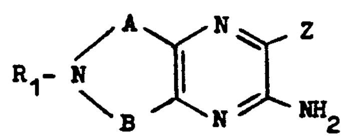

(XIII)

in der

$R_1$, A und B wie im Anspruch 1 definiert sind und

Z eine nukleophile Austrittsgruppe wie ein Halogenatom darstellt, mit einer Verbindung der allgemeinen Formel

$$H—R_2'' \quad (XIV)$$

in der

$R_2''$ eine Alkoxygruppe mit 1 bis 3 Kohlenstoffatomen darstellt, oder dessen Alkalisalz in einem Lösungsmittel umgesetzt wird und

gewünschtenfalls anschließend eine erfindungsgemäß erhaltene Verbindung der allgemeinen Formel I, in der $R_1$ eine gegebenenfalls durch eine Phenyl- oder Halogenphenylgruppe substituierte Alkanolgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Hydrolyse und gegebenenfalls anschließender Decarboxylierung in eine entsprechende Verbindung der allgemeinen Formel I, in der $R_1$ ein Wasserstoffatom darstellt, übergeführt wird und/oder

eine erhaltene Verbindung der allgemeinen Formel I, in der $R_1$ eine gegebenenfalls durch eine Phenyl- oder Halogenphenylgruppe substituierte Alkanoylgruppe mit 1 bis 3 Kohlenstoffatomen, eine Alkoxycarbonylgruppe mit insgesamt 2 bis 4 Kohlenstoffatomen oder eine Aralkoxycarbonylgruppe mit insgesamt 8 bis 10 Kohlenstoffatomen darstellt, mittels Reduktion in eine entsprechende Verbindung der allgemeinen Formel I, in der $R_1$ eine gegebenenfalls durch eine Phenyl- oder Halogenphenylgruppe substituierte Alkylgruppe mit 1 bis 3 Kohlenstoffatomen darstellt, übergeführt wird und/oder

eine erhaltene Verbindung der allgemeinen Formel I, in der $R_1$ eine gegebenenfalls durch ein

Halogenatom substituierte Benzylgruppe darstelle, durch Umsetzung mit Chlorameisensäurebenzylester und anschließend mit eisessig/Bromwasserstoff in eine entsprechende Verbindung der allgemein Formel I, in der $R_1$ ein Wasserstoffatom darstellt, übergeführt wird und/oder

eine erhaltene Verbindung der allgemeinen Formel I, in der $R_2$ ein Wasserstoffatom und $R_3$ eine Aminogruppe darstellt, mittels Halogenierung in eine entsprechende Verbindung der allgemeinen Formel I, in der $R_2$ ein Halogenatom darstellt, übergeführt wird und/oder

eine erhaltene Verbindung der allgemeinen Formel I in ihr Säureadditionssalz, insbesondere in ihr physiologisch verträgliches Salz mit einer anorganischen oder organischen Säure übergeführt wird.

8. Verfahren zur Herstellung von neuen Pyrazino-azepinen der allgemeinen Formel

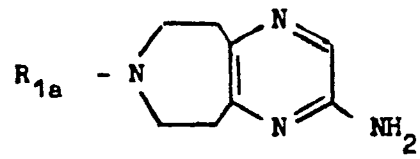

(Ia)

in der

$R_{1a}$ ein Wasserstoffatom, eine Alkylgruppe mit 1 bis 6 Kohlenstoffatomen, eine Alkenylgruppe mit 3 bis 6 Kohlenstoffatomen, eine Alkylgruppe mit 1 bis 3 Kohlenstoffatomen, welche durch eine gegebenenfalls durch ein Halogenatom substituierte Phenylgruppe substituiert ist, eine Cycloalkylgruppe mit 5 bis 7 Kohlenstoffatomen, eine Alkanoylgruppe mit 1 bis 3 Kohlenstoffatomen, eine Alkanoylgruppe mit 1 bis 3 Kohlenstoffatomen, eine Alkoxycarbonylgruppe mit insgesamt 2 bis 4 Kohlenstoffatomen, eine gegebenenfalls durch ein Halogenatom substituierte Benzoylgruppe oder die Phenylgruppe bedeutet, und von deren physiologisch verträglichen Säureadditionssalzen mit anorganischen oder organischen Säuren, dadurch gekennzeichnet, daß ein gegebenenfalls nicht gereinigtes Diketon der allgemeinen Formel

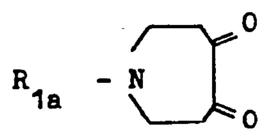

(IIa)

in der

$R_{1a}$ wie eingangs definiert ist, in einem Lösungsmittel mit 2-Amino-Acetamidin umgesetzt wird und

gewünschtenfalls anschließend eine erfindungsgemäß erhaltene Verbindung der allgemeinen Formel Ia, in der $R_{1a}$ eine Alkanoylgruppe mit 1 bis Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Hydrolyse und gegebenenfalls anschließender Decarboxylierung in die Verbindung der allgemeinen Formel Ia, in der $R_{1a}$ Wasserstoff darstellt, übergeführt wird und/oder

eine erhaltene Verbindung der allgemeinen Formel Ia, in der $R_{1a}$ eine Alkanoylgruppe mit 1 bis 3 Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Reduktion in eine Verbindung der allgemeinen Formel Ia, in der $R_{1a}$ eine Alkylgruppe mit 1 bis 3 Kohlenstoffatomen oder eine gegebenenfalls durch ein Halogenatom substituierte Benzylgruppe darstellt, übergeführt wird und/oder

eine erhaltene Verbindung der allgemeinen Formel Ia in ihre physiologisch verträglichen Säureadditionssalze mit anorganischen oder organischen Säuren übergeführt wird.

9. Verfahren zur Herstellung von neuen Pyrazino-azepinen der allgemeinen Formel Ia gemäß Anspruch 8 und von deren physiologisch verträglichen Säureadditionssalzen mit anorganischen oder organischen Säuren, dadurch gekennzeichnet, daß ein gegebenenfalls im Reaktionsgemisch gebildetes Pyrazin-N-oxid der allgemeinen Formel

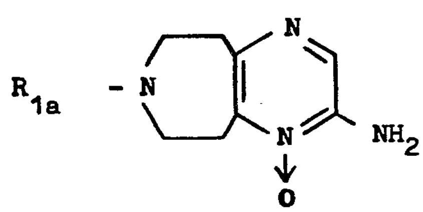

(IVa)

in der

$R_{1a}$ wie im Anspruch 8 definiert ist, in einem Lösungsmittel reduziert wird und

gewünschtenfalls anschließend eine erfindungsgemäß erhaltene Verbindung der allgemeinen Formel Ia, in der $R_{1a}$ eine Alkanoylgruppe mit 1 bis 3 Kohlenstoffatomen, eine Alkoxycarbonylgruppe mit

insgesamt 2 bis 4 Kohlenstoffatomen oder eine gegebenenfalls durch ein Halogenatom substituierte Benzoylgruppe darstellt, mittels Hydrolyse und gegebenenfalls anschließender Decarboxylierung in die Verbindung der allgemeinen Formel Ia, in der $R_{1a}$ Wasserstoff darstellt, übergeführt wird und/oder

eine erhaltene Verbindung der allgemeinen Formel Ia, in der $R_{1a}$ eine Alkanoylgruppe mit 1 bis 3 Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Reduktion in eine Verbindung der allgemeinen Formel Ia, in der $R_{1a}$ eine Alkylgruppe mit 1 bis 3 Kohlenstoffatomen oder eine gegebenenfalls durch ein Halogenatom substituierte Benzylgruppe darstellt, übergeführt wird und/oder

eine erhaltene Verbindung der allgemeinen Formel Ia in ihre physiologisch verträglichen Säureadditionssalze mit anorganischen oder organischen Säuren übergeführt wird.

10. Verfahren zur Herstellung von neuen Pyrazino-azepinen der allgemeinen Formel Ia gemäß Anspruch 8 und von deren physiologisch verträglichen Säureadditionssalzen mit anorganischen oder organischen Säuren, dadurch gekennzeichnet, daß eine gegebenenfalls im Reaktionsgemisch gebildete Verbindung der allgemeinen Formel

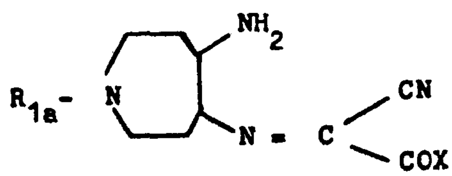

(Va)

in der
$R_{1a}$ wie im Anspruch 8 definiert ist und
X eine Alkoxygruppe darstellt, cyclisiert und eine so erhaltene Verbindung der allgemeinen Formel

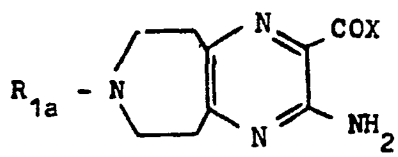

(VIa)

in der
$R_{1a}$ und X wie oben definiert sind, in einem Lösungsmittel hydrolysiert und decarboxyliert wird und gewünschtenfalls anschließend eine erfindungsgemäß erhaltene Verbindung der allgemeinen Formel Ia, in der $R_{1a}$ eine Alkanoylgruppe mit 1 bis 3 Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Hydrolyse und gegebenenfalls anschließender Decarboxylierung in die Verbindung der allgemeinen Formel Ia, in der $R_{1a}$ Wasserstoff darstellt, übergeführt wird und/oder

eine erhaltene Verbindung der allgemeinen Formel Ia, in der $R_{1a}$ eine Alkanoylgruppe mit 1 bis 3 Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Reduktion in eine Verbindung der allgemeinen Formel Ia, in der $R_{1a}$ eine Alkylgruppe mit 1 bis 3 Kohlenstoffatomen oder eine gegebenenfalls durch ein Halogenatom substituierte Benzylgruppe darstellt, übergeführt wird und/oder

eine erhaltene Verbindung der allgemeinen Formel Ia in ihre physiologisch verträglichen Säureadditionssalze mit anorganischen oder organischen Säuren übergeführt wird.

11. Verfahren zur Herstellung von neuen Pyrazino-azepinen der allgemeinen Formel Ia gemäß Anspruch 8 und von deren physiologisch verträglichen Säureadditionssalzen mit anorganischen oder organischen Säuren, dadurch gekennzeichnet, daß ein Pyrazino [2,3-d] azepin der allgemeinen Formel

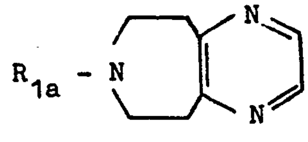

(VIIa)

in der
$R_{1a}$ wie im Anspruch 8 definiert ist, mit einem Alkaliamid in einem Lösungsmittel umgesetzt wird und gewünchtenfalls anschließend eine erfindungsgemäß erhaltene Verbindung der allgemeinen Formel Ia, in der $R_{1a}$ eine Alkanoylgruppe mit 1 bis 3 Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Hydrolyse und gegebenenfalls anschließender Decarboxylierung in die Verbindung der allgemeinen Formel Ia, in der $R_{1a}$ Wasserstoff darstellt, übergeführt wird und/oder

eine erhaltene Verbindung der allgemeinen Formel Ia, in der $R_{1a}$ eine Alkanoylgruppe mit 1 bis 3 Kohlenstoffatomen, eine Alkoxycarbonylgruppe mit insgesamt 2 bis 4 Kohlenstoffatomen oder eine gegebenenfalls durch ein Halogenatom substituierte Benzoylgruppe darstellt, mittels Reduktion in eine Verbindung der allgemeinen Formel Ia, in der $R_{1a}$ eine Alkylgruppe mit 1 bis 3 Kohlenstoffatomen oder eine gegebenenfalls durch ein Halogenatom substituierte Benzylgruppe darstellt, übergeführt wird und/oder

eine erhaltene Verbindung der allgemeinen Formel Ia in ihre physiologisch verträglichen säureadditionssalze mit anorganischen oder organischen Säuren übergeführt wird.

12. Verfahren gemäß Anspruch 5, dadurch gekennzeichnet, daß eine im Reaktionsgemisch gebildete Verbindung der allgemeinen Formel

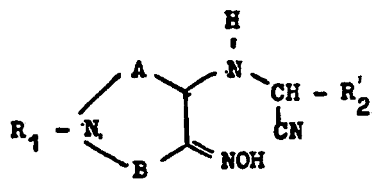

(X)

in der

$R_1$, $R_2'$, A und B wie im Anspruch 5 definiert sind, in Gegenwart eines sauren Kondensationsmittels cyclisiert wird.

**Claims** (for the Contracting States : BE, CH, DE, FR, IT, LI, LU, NL, SE)

1. New pyrazines of general formula

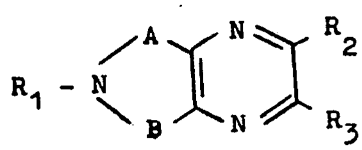

(I)

wherein

A and B, which may be the same or different, each represent a methylene or ethylene group optionally substituted by an alkyl group with 1 to 3 carbon atoms ;

$R_1$ represents a hydrogen atom ; an alkyl group with 4 to 6 carbon atoms ; a cycloalkyl group with 3 to 7 carbon atoms ; an alkenyl group with 3 to 6 carbon atoms ; an alkyl or alkanoyl group with 1 to 3 carbon atoms which may be substituted by a phenyl group optionally substituted by a halogen atom ; an alkoxycarbonyl group with a total of 2 to 4 carbon atoms ; an aralkoxycarbonyl group with a total of 8 to 10 carbon atoms or a phenyl group ;

one of the groups $R_2$ or $R_3$ represents an amino group and

the other group $R_2$ or $R_3$ represents a hydrogen, chlorine or bromine atom, an alkyl group with 1 to 3 carbon atoms, an alkoxy group with 1 to 3 carbon atoms, an alkoxycarbonyl group with a total of 2 to 4 carbon atoms, a phenyl or halophenyl group, and the acid addition salts thereof, particularly the physiologically acceptable salts with inorganic or organic acids.

2. New compounds of general formula I as claimed in claim 1, wherein

A and B, which may be the same or different, each represent a methylene, ethylene or methylethylene group,

$R_1$ represents a hydrogen atom, an alkyl group with 1 to 4 carbon atoms, a cycloalkyl group with 3 to 6 carbon atoms, an alkenyl group with 3 to 4 carbon atoms, an alkyl group with 1 to 3 carbon atoms substituted by a phenyl or chlorophenyl group, a formyl or acetyl group optionally substituted by a phenyl or chlorophenyl group, an ethoxycarbonyl or phenyl group.

$R_2$ represents a hydrogen, chlorine or bromine atom, a methyl, ethyl, methoxy, phenyl or ethoxycarbonyl group and

$R_3$ represents an amino group, and the acid addition salts thereof, more particularly the physiologically acceptable acid addition salts thereof with inorganic or organic acids.

3. New compounds of general formula I as claimed in claim 1, wherein

A represents a methylene or ethylene group,

B represents the ethylene group,

$R_1$ represents a methyl, ethyl, isopropyl, cyclopropyl, cyclobutyl or allyl group,

$R_2$ represents a methyl group, a hydrogen, chlorine or bromine atom and

$R_3$ represents an amino group, and the physiologically acceptable acid addition salts thereof with inorganic or organic acids.

4. New compounds of general formula I as claimed in claim 1, wherein
B, $R_1$ and $R_3$ are defined as in claim 3,
A represents the ethylene group and
$R_2$ represents a hydrogen atom, and the physiologically acceptable acid addition salts thereof with inorganic or organic acids.

5. 7-Ethyl-2-amino-6,7,8,9-tetrahydro-5H-pyrazino [2,3-d] azepine and the acid addition salts thereof.

6. 2-Amino-6,7,8,9-tetrahydro-7-methyl-5H-pyrazino [2,3-d] azepine and the acid addition salts thereof.

7. 6-Ethyl-2-amino-5,6,7,8-tetrahydro-pyrido [3,4]-pyrazine and the acid addition salts thereof.

8. Pharmaceutical compositions containing a compound as claimed in claims 1 to 7 together with one or more inert carriers or diluents.

9. Use of a compound as claimed in claims 1 to 7 for the preparation, by a non-chemical method, of a pharmaceutical product for treating cardiac and circulatory diseases.

10. Process for preparing new pyrazines of general formula I as claimed in claim 1 and the acid addition salts thereof, particularly the physiologically acceptable salts thereof with inorganic or organic acids, characterised in that, in order to prepare compounds of general formula I wherein one of the groups $R_2$ or $R_3$ represents a hydrogen atom, an alkyl group with 1 to 3 carbon atoms, an alkoxycarbonyl group with a total of 2 to 4 carbon atoms, a phenyl or halophenyl group and the other group $R_2$ or $R_3$ represents an amino group, a diketone of general formula

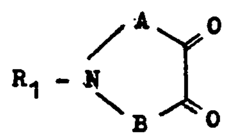

(II)

optionally formed in the reaction mixture wherein
A, B and $R_1$ are defined as in claim 1, is reacted with a 2-amino-acetamidine of general formula

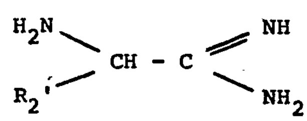

(III)

wherein
$R_2'$ represents a hydrogen atom, an alkyl group with 1 to 3 carbon atoms, an alkoxycarbonyl group with a total of 2 to 4 carbon atoms, a phenyl or halophenyl group, in a solvent, and
subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or
a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or
a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom
and/or a compound of general formula I obtained wherein $R_2$ represents a hydrogen atom and $R_3$ represents an amino group, is converted by halogenation into a corresponding compound of general formula I wherein $R_2$ represents a halogen atom, and/or
a compound of general formula I obtained is converted into the acid addition salt thereof, more particularly the physiologically acceptable salt thereof with an inorganic or organic acid.

11. Process for the preparation of new pyrazines of general formula I as claimed in claim 1 and of the acid addition salts thereof, particularly the physiologically acceptable salts with inorganic or organic acids, characterised in that a pyrazine-N-oxide of general formula

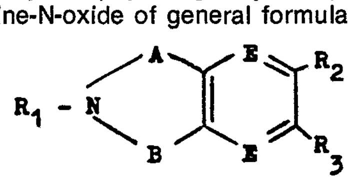

(IV)

optionally formed in the reaction mixture, wherein

$R_1$ to $R_3$, A and B A and B are as hereinbefore defined, one of the groups E represents an N-oxide group and the other group E represents a nitrogen atom or an N-oxide group, is reduced in a solvent, and subsequently, if desired, a compound of general fromula I obtained according to the invention, wherein $R_1$ represents an alkanoyl group with 1 to 3 carbon atoms optionally substituted by a phenyl or halophenyl group, an lakoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

12. Process for the preparation of new pyrazines of general formula I as claimed in claim 1 and of the acid addition salts thereof, particularly the physiologically acceptable salts with inorganic or organic acids, characterised in that, in order to prepare compounds of general formula I wherein one of the groups $R_2$ or $R_3$ represents an amino group and the other group $R_2$ or $R_3$ represents a hydrogen atom or an alkoxycarbonyl group with a total of 2 to 4 carbon atoms, a compound of general formula

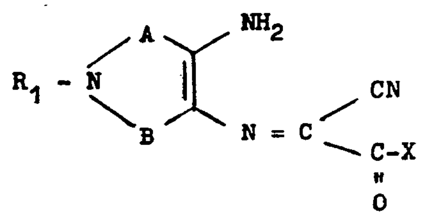

(V)

optionally formed in the reaction mixture, wherein

$R_1$, A and B are defined as in claim 1 and

X represents an alkoxy group, is cyclised and, if desired, a compound of general formula

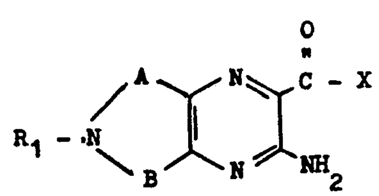

(VI)

thus obtained, wherein

$R_1$, A, B and X are as hereinbefore defined, is hydrolysed and decarboxylated, and

subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reudction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

13. Process for the preparation of new pyrazines of general formula I as claimed in claim 1 and of the acid addition salts thereof, particularly the physiologically acceptable salts with inorganic or organic acids, characterised in that, in order to prepare compounds of general formula I in which one of the groups $R_2$ or $R_3$ represents an amino group and the other group $R_2$ or $R_3$ represents a hydrogen atom, a pyrazine of general formula

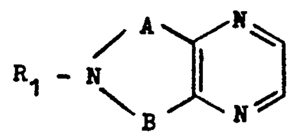

(VII)

wherein

$R_1$, A and B are defined as in claim 1, is reacted with an alkali metal amide, and subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

14. Process for the preparation of new pyrazines of general formula I as claimed in claim 1 and of the acid addition salts thereof, particularly the physiologically acceptable salts with inorganic or organic acids, characterised in that, in order to prepare compounds of general formula I wherein one of the groups $R_2$ or $R_3$ represents an amino group and the other group $R_2$ or $R_3$ represents a hydrogen atom, an alkyl group with 1 to 3 carbon atoms, an alkoxycarbonyl group with a total of 2 to 4 carbon atoms, or a phenyl or halophenyl group, an oxime of general formula

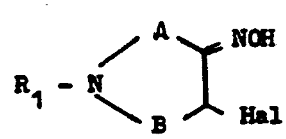

(VIII)

optionally formed in the reaction mixture, wherein

$R_1$, A and B are defined as in claim 1 and

Hal represents a chlorine, bromine or iodine atom, is reacted with a 2-amino-acetonitrile of general formula

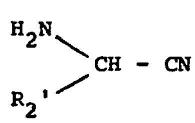

(IX)

wherein

$R_2'$ represents a hydrogen atom, an alkyl group with 1 to 3 carbon atoms, an alkoxycarbonyl group with a total of 2 to 4 carbon atoms, an alkoxycarbonyl group with a total of 2 to 4 carbon atos, a phenyl or halophenyl group, in a solvent, and

subsequently, if desired, a compound of general formula I obtained according to the invention,

wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 tp 10 carbon atoms, is subsequently converted, by hydrolysis and optionally subsequent decarboxylation, into a corresponding compound of general formula I, wherein $R_1$ represents a hydrogen atom, and/or

a compound of general formula I obtained wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

15. Process for the preparation of new pyrazines of general formula I as claimed in claim 1 and of the acid addition salts thereof, particularly the physiologically acceptable salts with inorganic or organic acids, characterised in that, in order to prepate compounds of general formula I wherein $R_1$ has the meanings given for $R_1$ in claim 1 with the exception of the hydrogen atom and the phenyl group, a pyrazine of general formula

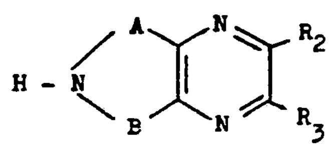

(XI)

wherein

$R_2$, $R_3$, A and B are defined as in claim 1, is reacted with a compound of general formula

$$R_1'—Y \quad (XII)$$

wherein

$R_1'$ has the definitions given for $R_1$ hereinbefore, with the exception of the hydrogen atom and the phenyl group, and

Y represents a nucleophilic leaving group, and

subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acidaddition salt thereof, more particularly the physiologically acceptable salt thereof with an inorganic or organic acid.

16. Process for the preparation of new pyrazines of general formula I as claimed in claim 1 and of the acid addition salts thereof, particularly the physiologically acceptable salts with inorganic or organic acids, characterised in that, in order to prepare compounds of general formula I wherein one of the groups $R_2$ or $R_3$ represent an amino group and the other group $R_2$ or $R_3$ represents an alkoxy group with 1 to 3 carbon atoms, a pyrazine of general formula XIII

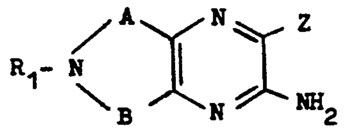

wherein

$R_1$, A and B are defined as in claim 1 and

Z represents a nucleophilic leaving group such as a halogen atom, is reacted with a compound of general formula

$$H—R_2'' \qquad (XIV)$$

wherein

$R_2''$ represents an alkoxy group with 1 to 3 carbon atoms, or the alkali salt thereof, in a solvent and subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

**Claims** (for the Contracting State AT)

1. Process for the preparation of new pyrazines of general formula

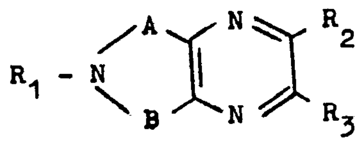

(I)

wherein

A and B, which may be the same or different, each represent a methylene or ethylene group optionally substituted by an alkyl group with 1 to 3 carbon atoms ;

$R_1$ represents a hydrogen atom ; an alkyl group with 4 to 6 carbon atoms ; a cycloalkyl group with 3 to 7 carbon atoms ; an alkenyl group with 3 to 6 carbon atoms ; an alkyl or alkanoyl group with 1 to 3 carbon atoms which may be substituted by a phenyl group optionally substituted by a halogen atom ; an alkoxycarbonyl group with a total of 2 to 4 carbon atoms ; an aralkoxycarbonyl group with a total of 8 to carbon atoms or a phenyl group ;

one of the groups $R_2$ or $R_3$ represents an amino group and

the other group $R_2$ or $R_3$ represents a hydrogen, chlorine or bromine atom, an alkyl group with 1 to 3 carbon atoms, an alkoxy group with 1 to 3 carbon atoms, an alkoxycarbonyl group with a total of 2 to 4 carbon atoms, a phenyl or halophenyl group, and the acid addition salts thereof, particularly the physiologically acceptable salts with inorganic or organic acids, characterised in that in order to prepare compounds of general formula I wherein one of the groups $R_2$ or $R_3$ represents a hydrogen atom, an alkyl group with 1 to 3 carbon atoms, an alkoxycarbonyl group with a total of 2 to 4 carbon atoms, a phenyl or halophenyl group and

the other group $R_2$ or $R_3$ represents an amino group, a diketone of general formula

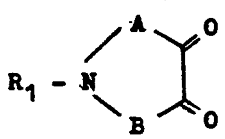

(II)

optionally formed in the reaction mixture wherein

A, B and $R_1$ are defined as in claim 1, is reacted with a 2-amino-acetamidine of general formula

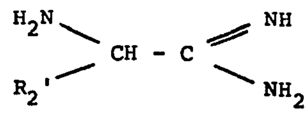

(III)

wherein

$R_2'$ represents a hydrogen atom, an alkyl group with 1 to 3 carbon atoms, an alkoxycarbonyl group with a total of 2 to 4 carbon atoms, a phenyl or halophenyl group, in a solvent, and

subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

2. Process for the preparation of new pyrazines of general formula I as claimed in claim 1 and of the acid addition salts thereof, particularly the physiologically acceptable salts with inorganic or organic acids, characterised in that a pyrazine-N-oxide of general formula

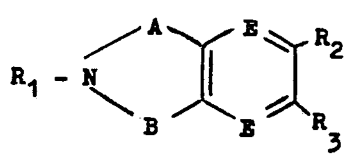

(IV)

optionally formed in the reaction mixture, wherein

$R_1$ to $R_3$, A and B are as hereinbefore defined, one of the groups E represents an N-oxide group and the other group E represents a nitrogen atom or an N-oxide group, is reduced in a solvent, and subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

3. Process for the preparation of new pyrazines of general formula I as claimed in claim 1 and of the

acid addition salts thereof, particularly the physiologically acceptable salts with inorganic or organic acids, characterised in that, in order to prepare compounds of general formula I wherein one of the groups $R_2$ or $R_3$ represents an amino group and the other group $R_2$ or $R_3$ represents a hydrogen atom or an alkoxycarbonyl group with a total of 2 to 4 carbon atoms, a compound of general formula

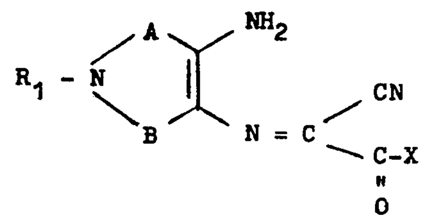

(V)

optionally formed in the reaction mixture, wherein

$R_1$, A and B are defined as in claim 1 and

X represents an alkoxy group, is cyclised and, if desired, a compound of general formula

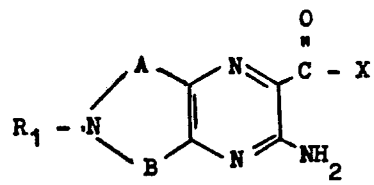

(VI)

thus obtained, wherein

$R_1$, A, B and X are as hereinbefore defined, is hydrolysed and decarboxylated, and

subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid-hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

4. Process for the preparation of new pyrazines of general formula I as claimed in claim 1 and of the acid addition salts thereof, particularly the physiologically acceptable salts with inorganic or organic acids, characterised in that, in order to prepare compounds of general formula I in which one of the groups $R_2$ or $R_3$ represents an amino group and the other group $R_2$ or $R_3$ represents a hydrogen atom, a pyrazine of general formula

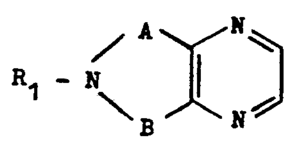

(VII)

wherein

$R_1$, A and B are defined as in claim 1, is reacted with an alkali metal amide, and subsequently, if desired, a compound of general formula I obtained

according to the invention, wherein $R_1$ represents an alkanoyl group with 1 to 3 carbon atoms optionally substituted by a phenyl or halophenyl group, an alkoxycarbonyl group with a total of 2 to 4

carbon atoms or an aralkoxycarbonyl group with a total of 8 to 10 carbon atoms, is subsequently converted, by hydrolysis and optionally subsequent decarboxylation, intoo a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

5. Process for the preparation of new pyrazines of general formula I as claimed in claim 1 and of the acid addition salts thereof, particularly the physiologically acceptable salts with inorganic or organic acids, characterised in that, in order to prepare compounds of general formula I wherein one of the groups $R_2$ or $R_3$ represents an amino group and the other group $R_2$ or $R_3$ represents a hydrogen atom, an alkyl group with 1 to 3 carbon atoms, an alkoxycarbonyl group with a total of 2 to 4 carbon atoms, or a phenyl or halophenyl group, an oxime of general formula

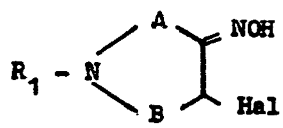

(VIII)

optionally formed in the reaction mixture, wherein

$R_1$, A and B are defined as in claim 1 and

Hal represents a chlorine, bromine or iodine atom, is reacted with a 2-amino-acetonitrile of general formula

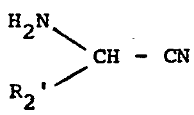

(IX)

wherein

$R_2'$ represents a hydrogen atom, an alkyl group with 1 to 3 carbon atoms, an alkoxycarbonyl group with a total of 2 to 4 carbon atoms, an alkoxycarbonyl group with a total of 2 to 4 carbon atos, a phenyl or halophenyl group, in a solvent, and

subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of geneaal formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

6. Process for the preparation of new pyrazines of general formula I as claimed in claim 1 and of the acid addition salts thereof, particularly the physiologically acceptable salts with inorganic or organic

acids, characterised in that, in order to prepare compounds of general formula I wherein $R_1$ has the meanings given for $R_1$ in claim 1 with the exception of the hydrogen atom and the phenyl group, a pyrazine of general formula

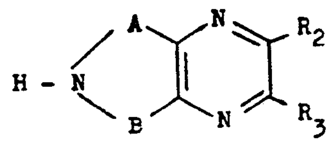

(XI)

wherein

$R_2$, $R_3$, A and B are defined as in claim 1, is reacted with a compound of general formula

$$R_1'—Y \qquad (XII)$$

wherein

$R_1'$ has the definitions given for $R_1$ hereinbefore, with the exception of the hydrogen atom and the phenyl group, and

Y represents a nucleophilic leaving group, and

subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

7. Process for the preparation of new pyrazines of general formula I as claimed in claim 1 and of the acid addition salts thereof, particularly the physiologically acceptable salts with inorganic or organic acids, characterised in that, in order to prepare compounds of general formula I wherein one of the groups $R_2$ or $R_3$ represent an amino group and the other group $R_2$ or $R_3$ represents an alkoxy group with 1 to 3 carbon atoms,

a pyrazine of general formula XIII

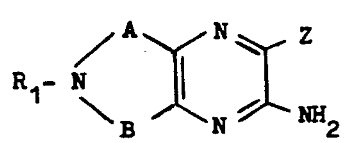

(XIII)

wherein

$R_1$, A and B are defined as in claim 1 and

Z represents a nucleophilic leaving group such as a halogen atom, is reacted with a compound of general formula

$$H—R_2'' \qquad (XIV)$$

wherein

$R_2''$ represents an alkoxy group with 1 to 3 carbon atoms, or the alkali salt thereof, in a solvent and subsequently, if desired, a compound of general formula I obtained according to the invention, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, is subsequently converted, by hydrolysis and optionally

subsequent decarboxylation, into a corresponding compound of general formula I, wherein $R_1$ represents a hydrogen atom, and/or

a compound of general formula I obtained, wherein $R_1$ represents an alkanoyl group with 1 to 3 carbon atoms optionally substituted by a phenyl or halophenyl group, an alkoxycarbonyl group with a total of 2 to 4 carbon atoms or an aralkoxycarbonyl group with a total of 8 to 10 carbon atoms, may be converted by reduction into a corresponding compound of general formula I wherein $R_1$ represents an alkyl group with 1 to 3 carbon atoms optionally substituted by a phenyl or halophenyl group, and/or

a compound of general formula I obtained wherein $R_1$ represents a benzyl group optionally substituted by a halogen atom, is converted, by reaction with benzyl chloroformate and then with glacial acetic acid/hydrogen bromide, into a corresponding compound of general formula I wherein $R_1$ represents a hydrogen atom,

and/or a compound of general formula I obtained wherein $R_2$ represents a hydrogen atom and $R_3$ represents an amino group, is converted by halogenation into a corresponding compound of general formula I wherein $R_2$ represents a halogen atom, and/or

a compound of general formula I obtained is converted into the acid addition salt thereof, more particularly the physiologically acceptable salt thereof with an inorganic or organic acid.

8. Process for the preparation of new pyrazino-azepines of general formula

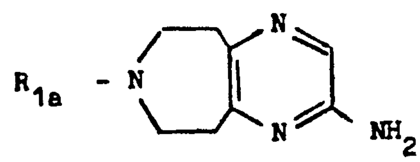

(Ia)

wherein

$R_{1a}$ represents a hydrogen atom, an alkyl group with 1 to 6 carbon atoms, an alkenyl group with 3 to 6 carbon atoms, an alkyl group with 1 to 3 carbon atoms which is substituted by a phenyl group optionally substituted by a halogen atom, a cycloalkyl group with 5 to 7 carbon atoms, an alkylanoyl group with 1 to 3 carbon atoms, an alkoxycarbonyl group with a total of 2 to 4 carbon atoms, a benzoyl group optionally substituted by a halogen atom or a phenyl group, and the physiologically acceptable acid addition salts thereof with inorganic or organic acids, characterised in that an optionally unpurified diketone of general formula

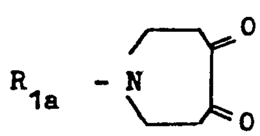

(IIa)

wherein

$R_{1a}$ is as hereinbefore defined, is reacted in a solvent with 2-amino-acetamidine and

subsequently, if desired, a compound of general formula Ia obtained according to the invention, wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by hydrolysis and optionally subsequent decarboxylation into a compound of general formula Ia wherein $R_{1a}$ represents hydrogen, and/or

a compound of general formula Ia obtained, wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by reduction into a compound of general formula Ia wherein $R_{1a}$ represents an alkyl group with 1 to 3 carbon atoms or a benzyl group optionally substituted by a halogen atom, and/or

a compound of general formula Ia obtained is converted into the physiologically acceptable acid addition salts thereof with inorganic or organic acids.

9. Process for the preparation of new pyrazino-azepines of general formula Ia as claimed in claim 8 and of the physiologically acceptable acid addition salts thereof with inorganic or organic acids, characterised in that a pyrazine-N-oxide of general formula

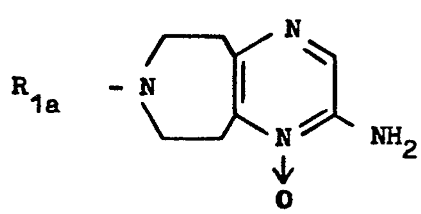

(IVa)

optionally prepared in the reaction mixture, wherein

$R_{1a}$ is defined as in claim 8, is reduced in a solvent, and

subsequently, if desired, a compound of general formula Ia obtained according to the invention wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by hydrolysis and optionally subsequent decarboxylation into a compound of general formula Ia wherein $R_{1a}$ represents hydrogen, and/or

a compound of general formula Ia obtained, wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by reduction into a compound of general formula Ia wherein $R_{1a}$ represents an alkyl group with 1 to 3 carbon atoms or a benzyl group optionally substituted by a halogen atom, and/or

a compound of general formula Ia obtained is converted into the physiologically acceptable acid addition salts thereof with inorganic or organic acids.

10. Process for the preparation of new pyrazino-azepines of general formula Ia as claimed in claim 8 and of the physiologically acceptable acid addition salts thereof with inorganic or organic acids, characterised in that a compound of general formula (Va)

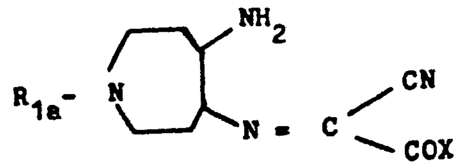

(Va)

optionally formed in the reaction mixture, wherein

$R_{1a}$ is defined as in claim 8 and

X is an alkoxy group, is cyclised and a compound of general formula

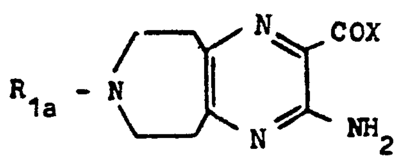

(VIa)

thus obtained wherein

$R_{1a}$ and X are as hereinbefore defined, is hydrolysed and decarboxylated in a solvent, and

subsequently, if desired, a compound of general formula Ia obtained according to the invention, wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by hydrolysis and optionally subsequent decarboxylation into a compound of general formula Ia wherein $R_{1a}$ represents hydrogen, and/or

a compound of general formula Ia obtained, wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by reduction into a compound of general formula Ia wherein $R_{1a}$ represents an alkyl group with 1 to 3 carbon atoms or a benzoyl group optionally substituted by a halogen atom, and/or

a compound of general formula Ia obtained is converted into the physiologically acceptable acid addition salts thereof with inorganic or organic acids.

11. Process for the preparation of new pyrazino-azepines of general formula Ia as claimed in claim 8 and of the physiologically acceptable acid addition salts thereof with inorganic or organic acids, characterised in that a pyrazino [2,3-d] azepine of general formula

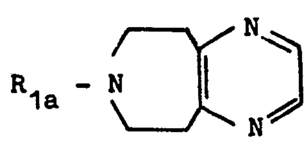

(VIIa)

wherein

$R_{1a}$ is defined as in claim 8, is reacted with an alkali metal amide in a solvent, and

subsequently, if desired, a compound of general formula Ia obtained according to the invention, wherein $R_{1a}$ represents an alkanoyl group with 1 to 3 carbon atoms, an alkoxycarbonyl group with a total of 2 to 4 carbon atoms or a benzoyl group optionally substituted by a halogen atom, is converted by

hydrolysis and optionally subsequent decarboxylation into a compound of general formula Ia wherein $\bar{R}_{1a}$ represents hydrogen, and/or

a compound of general formula Ia obtained, wherein $R_{1a}$ represents an alkanoyl group with 1 to 3 carbon atoms, an alkocycarbonyl group with a total of 2 to 4 carbon atoms or a benzoyl group optionally substituted by a halogen atom, is converted by reduction into a compound of general formula Ia wherein $R_{1a}$ represents an alkyl group with 1 to 3 carbon atoms or a benzyl group optionally substituted by a halogen atom, and/or

a compound of general formula Ia obtained is converted into the physiologically acceptable acid addition salts thereof with inorganic or organic acids.

12. Process as claimed in claim 5, characterised in that a compound of general formula

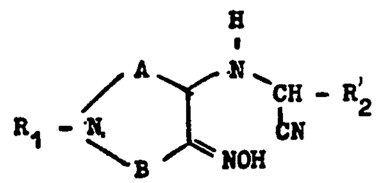

(X)

formed in the reaction mixture wherein

$R_1$, $R_2'$, A and B are defined as in claim 5, is cyclised in the presence of an acidic condensing agent.

**Revendications** (pour les Etats contractants : BE, CH, DE, FR, IT, LI, LU, NL, SE)

1. Nouvelles pyrazines de formule générale

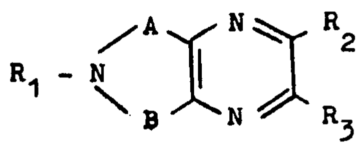

(I)

dans laquelle

A et B, qui peuvent être identiques ou différents, représentent chacun un groupe méthylène ou éthylène éventuellement substitué par un groupe alcoyle avec 1 à 3 atomes de carbone,

$R_1$ représente un atome d'hydrogène, un groupe alcoyle avec 4 à 6 atomes de carbone, un groupe cycloalcoyle avec 3 à 7 atomes de carbone, un groupe alcényle avec 3 à 6 atomes de carbone, un groupe alcoyle ou alcanoyle avec à chaque fois 1 à 3 atomes de carbone qui peut dans chaque cas être substitué par un groupe phényle éventuellement substitué par un atome d'halogène, ou représente un groupe alcoxycarbonyle avec en tout 2 à 4 atomes de carbone, un groupe aralcoxycarbonyle avec en tout 8 à 10 atomes de carbone ou le groupe phényle,

l'un des radicaux $R_2$ ou $R_3$ représente un groupe amino et l'autre des radicaux $R_2$ ou $R_3$ représente un atome d'hydrogène, de chlore ou de brome, un groupe alcoyle avec 1 à 3 atomes de carbone,

un groupe alcoxy avec 1 à 3 atomes de carbone, un groupe alcoxy-carbonyle avec en tout 2 à 4 atomes de carbone, un groupe phényle ou halogénophényle, et leurs sels d'addition d'acides, en particulier leurs sels physiologiquement supportables avec des acides minéraux ou organiques.

2. Nouveaux composés de formule générale I selon la revendication 1, dans laquelle

A et B qui peuvent être identiques ou différents représentent chacun un groupe méthylène, éthylène ou méthyléthylène,

$R_1$ représente un atome d'hydrogène, un groupe alcoyle avec 1 à 4 atomes de carbone, un groupe cycloalcoyle avec 3 à 6 atomes de carbone, un groupe alcényle avec 3 ou 4 atomes de carbone, un groupe alcoyle avec 1 à 3 atomes de carbone substitué par un groupe phényle ou chlorophényle, ou représente un groupe formyle ou acétyle avéntuellement substitué par un groupe phényle ou chlorophényle, ou représente un groupe éthoxycarbonyle ou phényle,

$R_2$ représente un atome d'hydrogène, de chlore ou de brome, un groupe méthyle, éthyle, méthoxy, phényle ou éthoxycarbonyle et

$R_3$ représente un groupe amino, et leurs sels d'addition d'acides, en particulier leurs sels physiologiquement supportables avec des acides minéraux ou organiques.

3. Nouveaux composés de formule générale I selon la revendication 1, dans laquelle

A représente un groupe méthylène ou éthylène,

B représente le groupe éthylène,

$R_1$ représente un groupe méthyle, éthyle, isopropyle, cyclopropyle, cyclobutyle ou allyle,

$R_2$ représente un groupe méthyle, un atome d'hydrogène, de chlore ou de brome et

$R_3$ représente un groupe amino, et leurs sels d'addition d'acides physiologiquement supportables avec des acides minéraux ou organiques.

4. Nouveaux composés de formule générale I selon la revendication 1, dans laquelle
B, $R_1$ et $R_3$ sont définis comme dans la revendication 3,
A représente le groupe éthylène et
$R_2$ représente un atome d'hydrogène, et leurs sels d'addition d'acides physiologiquement supportables avec des acides minéraux ou organiques.

5. La 7-éthyl-2-amino-6,7,8,9-tétrahydro-5H-pyrazino [2,3-d] azépine et ses sels d'addition d'acides.

6. La 2-amino-6,7,8,9-tétrahydro-7-méthyl-5H-pyrazino [2,3-d] azépine et ses sels d'addition d'acides.

7. La 6-éthyl-2-amino-5,6,7,8-tétrahydro-pyrido [3,4-b] pyrazine et ses sels d'addition d'acides.

8. Médicament contenant un composé selon les revendications 1 à 7 conjointement à un ou plusieurs excipients ou diluants inertes.

9. Utilisation d'un composé selon les revendications 1 à 7 pour la préparation d'un médicament par voie non chimique pour le traitement des maladies du cœur et du système circulatoire.

10. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I, dans laquelle l'un des radicaux $R_2$ ou $R_3$ représente un atome d'hydrogène, un groupe alcoyle avec 1 à 3 atomes de carbone, un groupe alcoxycarbonyle avec en tout 2 à 4 atomes de carbone, un groupe phényle ou halogénophényle et l'autre des radicaux $R_2$ ou $R_3$ représente un groupe amino, on fait réagir une dicétone éventuellement formée dans le mélange réactionnel, de formule générale

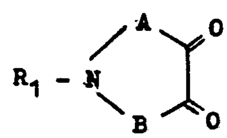

(II)

dans laquelle
A, B et $R_1$ sont définis comme dans la revendication 1, avec une 2-amino-acétamidine de formule générale

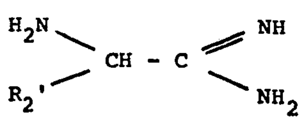

(III)

dans laquelle
$R_2'$ représente un atome d'hydrogène, un groupe alcoyle avec 1 à 3 atomes de carbone, un groupe alcoxy-carbonyle avec en tout 2 à 4 atomes de carbone, un groupe phényle ou halogénophényle, dans un solvant et
si on le désire ensuite on transforme un composé obtenu selon l'invention, de formule générale I dans laquelle $R_1$ représente un groupe alcanoyle avec 1 à 3 atomes de carbone éventuellement substitué par un groupe phénye ou halogénophényle, ou représente un groupe alcoxy-caabonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou
on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou
on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou
on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou
on transforme un composé obtenu de formule générale I en son sel d'addition d'acide, en particulier en son sel physiologiquement supportable avec un acide minéral ou organique.

11. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce qu'on réduit un pyrazine-N-oxyde éventuellement formé dans le mélange réactionnel, de formule générale

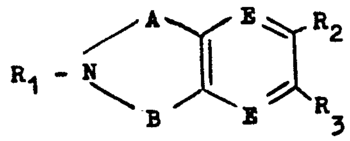

(IV)

dans laquelle

$R_1$ à $R_3$, A et B sont définis comme dans la revendication 1, l'un des radicaux E représente un groupe N-oxyde et l'autre des radicaux E représente un atome d'azote ou un groupe N-oxyde, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'ahlogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'ahlogène et/ou

on transforme un composé obtenu de formule générale I en son sel d'addition d'acide, en particulier en son sel physiologiquement supportable avec un acide minéral ou organique.

12. Procédé pour la préparation de nouvelles pyrazines de formule générale I selon la revendication 1 et de leurs sels d'addition d'acides, en particulier de leurs sels physiologiquement supportables avec des aicdes minéraux ou organique, caractérisé en ce que, pour la préparation de composés de formule générale I dans laquelle l'un des radicaux $R_2$ ou $R_3$ représente un groupe amino et l'autre des radicaux $R_2$ ou $R_3$ représente un atome d'hydrogène ou un groupe alcoxycarbonyle avec en tout 2 à 4 atomes de carbone, on cyclise un composé éventuellement formé dans le mélange réactionnel, de formule générale

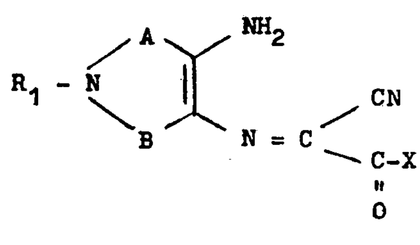

(V)

dans laquelle

$R_1$, A et B sont définis comme dans la revendication 1 et

X représente un groupe alcoxy, dans un solvant et si on le désire on hydrolyse et décarboxyle un composé ainsi obtenu de formule générale

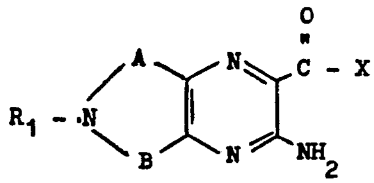

(VI)

dans laquelle

$R_1$, A, B et X sont définis comme plus haut et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone,

au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laqueller$_2$ représente un atome d'halogène et/ou

on transforme un composé obtenu de formule générale I en son sel d'addition d'acide, en particulier en son sel physiologiquement supportable avec un acide minéral ou organique.

13. Procédé pour la préparation de nouvelles pyrazines de formule générale I selon la revendication I et de leurs sels d'addition d'acides, en particulier de leurs sels physiologiquement supportables avec des acides minéraux ou organiques, caractérisé en ce que, pour la préparation de composé de formule générale I dans laquelle l'un des radicaux $R_2$ ou $R_3$ représente un groupe amino et l'autre des radicaux $R_2$ ou $R_3$ représente un atome d'hydrogène, on fait réagir une pyrazine de formule générale

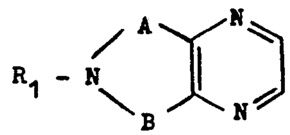

(VII)

dans laquelle

$R_1$, A et B sont définis comme dans la revendication 1, dans un solvant avec un amidure alcalin et si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son d'addition d'acide, en particulier en son sel physiologiquement supportable avec un acide minéral ou organique.

14. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérise en ce que, pour la préparation de composés de formule générale I dans laquelle l'un des radicaux $R_2$ ou $R_3$ représente un groupe amino et l'autre des radicaux $R_2$ ou $R_3$ représente un atome d'hydrogène, un groupe alcoyle avec 1 à 3 atomes de carbone, un groupe alcoxy-carbonyle avec en tout 2 à 4 atomes de carbone, un groupe phényle ou halogénophényle, on fait réagir une oxime éventuellement formée dans le mélange réactionnel, de formule générale

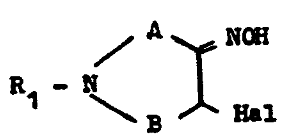

(VIII)

dans laquelle

$R_1$, A et B sont définis comme dans la revendication 1 et

Hal représente un atome de chlore, de brome ou d'iode, avec un 2-amino-acétonitrile de formule générale

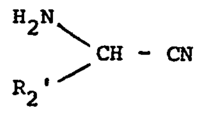

(IX)

dans laquelle

$R_2'$ représente un atome d'hydrogène, un groupe alcoyle avec 1 à 3 atomes de carbone, un groupe alcoxy-carbonyle avec en tout 2 à 4 atomes de carbone, un groupe phényle ou halogénophényle, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou hélogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

15. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I dans laquelle $R_1$ possède les significations mentionnées dans la revendication 1 pour $R_1$ à l'exception de l'atome d'hydrogène et du groupe phényle, on fait réagir une pyrazine de formule générale

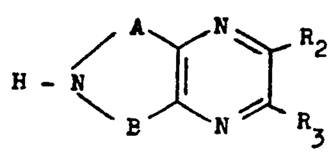

(XI)

dans laquelle

$R_2$, $R_3$, A et B sont définis comme dans la revendication 1, avec un composé de formule générale

$$R_1'—Y \quad (XII)$$

dans laquelle

$R_1'$ possède les significations mentionnées dans la revendication 1 pour $R_1$ à l'exception de l'atome d'hydrogène et du groupe phényle et

Y représente un groupe partant nucléophile, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un

composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

16. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I dans laquelle l'un des radicaux $R_2$ ou $R_3$ représente un groupe amino et l'autre des radicaux $R_2$ ou $R_3$ représente un groupe alcoxy avec 1 à 3 atomes de carbone, on fait réagir une pyrazine de formule générale

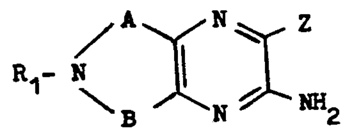

(XIII)

dans laquelle

$R_1$, A et B sont définis comme dans la revendication 1 et

Z représente un groupe partant nucléophile tel qu'un atome d'halogène, avec un composé de formule générale

$$H—R_2'' \qquad (XIV)$$

dans laquelle

$R_2''$ représente un groupe alcoxy avec 1 à 3 atomes de carbone, ou son sel alcalin dans un solvant et si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de bzneyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino-au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

**Revendications** (pour l'Etat contractant AT)

1. Procédé pour la préparation de nouvelles pyrazines de formule générale

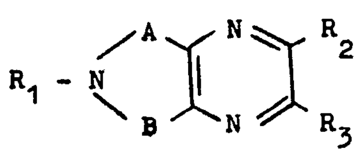

(I)

dans laquelle

A et B, qui peuvent être identiques ou différents, représentent chacun un groupe méthylène ou éthylène éventuellement substitué par un groupe alcoyle avec 1 à 3 atomes de carbone,

$R_1$ représente un atome d'hydrogène, un groupe alcoyle avec 4 à 6 atomes de carbone, un groupe cycloalcoyle avec 3 à 7 atomes de carbone, un groupe alcènyle avec 3 à 6 atomes de carbone, un groupe alcoyle ou alcanoyle avec à chaque fois 1 à 3 atomes de carbone qui peut dans chaque cas être substitué par un groupe phényle éventuellement substitué par un atome d'halogène ou représente un groupe alcoxycarbonyle avec en tout 2 à 4 atomes de carbone, un groupe aralcoxycarbonyle avec en tout 8 à 10 atomes de carbone, un groupe aralcoxycarbonyle avec en tout 8 à 10 atomes de carbone ou le groupe phényle,

l'un des radicaux $R_2$ ou $R_3$ représente un groupe amino et l'autre des radicaux $R_2$ ou $R_3$ représente un atome d'hydrogène, de chlore ou de brome, un groupe alcoyle avec 1 à 3 atomes de carbone,

un groupe alcoxy avec 1 à 3 atomes de carbone, un groupe alcoxy-carbonyle avec en tout 2 à 4 atomes de carbone, un groupe phényle ou halogénophényle, et de leurs sels d'addition d'acides, en particulier de leurs sels physiologiquement supportables avec des acides minéraux ou organiques, caractérisé en ce que, pour la préparation de composés de formule générale I, dans laquelle l'un des radicaux $R_2$ ou $R_3$ représente un atome d'hydrogène, un groupe alcoyle avec 1 à 3 atomes de carbone, un groupe alcoxycarbonyle avec en tout 2 à 4 atomes de carbone, un groupe phényle ou halogénophényle et

l'autre des radicaux $R_2$ ou $R_3$ représente un groupe amino, on fait réagir une dicatone éventuellement formée dans le mélange réactionnel, de formule générale

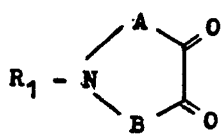

(II)

dans laquelle

A, B et $R_1$ sont définis comme au début, avec une 2-amino-acétamidine de formule générale

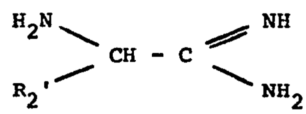

(III)

dans laquelle

$R_2'$ représente un atome d'hydrogène, un groupe alcoyle avec 1 à 3 atomes de carbone, un groupe alcoxy-carbonyle avec en tout 2 à 4 atomes de carbone, un groupe phényle ou halogénophényle, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

2. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce qu'on réduit un pyrazine-N-oxyde éventuellement formé dans le mélange réactionnel, de formule générale

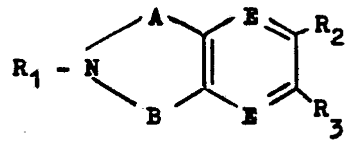

(IV)

dans laquelle

$R_1$ à $R_3$, A et B sont définis comme dans la revendication 1, l'un des radicaux E représente un groupe N-oxyde et l'autre des radicaux E représente un atome d'azote ou un groupe N-oxyde, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $r_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou)

on transforme un composé obtenu de formule générale I en son sel d'addition d'acide, en particulier en son sel physiologiquement supportable avec un acide minéral ou organique.

3. Procédé pour la préparation de nouvelles pyrazines de formule générale I selon la revendication 1 et de leurs sels d'addition d'acides, en particulier de leurs sels physiologiquement supportables avec des acides minéraux ou organique, caractérisé en ce que, pour la préparation de composés de formule générale I dans laquelle l'un des radicaux $R_2$ ou $R_3$ représente un groupe amino et l'autre des radicaux $R_2$ ou $R_3$ représente un atome d'hydrogène ou un groupe alcoxycarbonyle avec en tout 2 à 4 atomes de carbone, on cyclise un composé éventuellement formé dans le mélange réactionnel, de formule générale

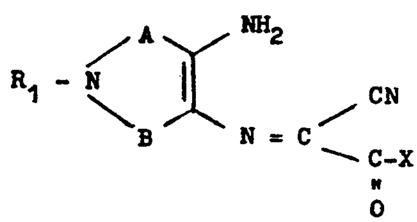

(V)

dans laquelle

$R_1$, A et B sont définis comme dans la revendication 1 et

X représente un groupe alcoxy, dans un solvant et si on le désire on hydrolyse et décarboxyle un composé ainsi obtenu de formule générale

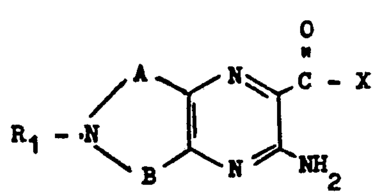

(VI)

dans laquelle

$R_1$, A, B et X sont définis comme plus haut et si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'aciee acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

4. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I dans laquelle l'un des radicaux $R_2$ ou $R_3$ représente un groupe amino et l'autre des radicaux $R_2$ ou $R_3$ représente un atome d'hydrogène, on fait réagir une pyrazine de formule générale

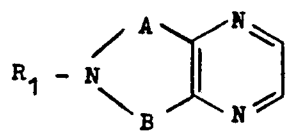

(VII)

dans laquelle

$R_1$, A et B sont définis comme dans la revendication 1, dans un solvant avec un amidure alcalin et si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

5. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I dans laquelle l'un des radicaux $R_2$ ou $R_3$ représente un groupe amino et l'autre des radicaux $R_2$ ou $R_3$ représente un atome d'hydrogène, un groupe alcoyle avec 1 à 3 atomes de carbone, un groupe alcoxy-carbonyle avec en tout 2 à 4 atomes de carbone, un groupe phényle ou halogénophényle, on fait réagir une oxime éventuellement formée dans le mélange réactionnel, de formule générale

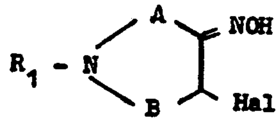

(VIII)

dans laquelle

$R_1$, A et B sont définis comme dans la revendication 1 et

Hal représente un atome de chlore, de brome ou d'iode, avec un 2-amino-acétonitrile de formule générale

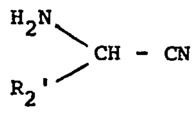

(IX)

dans laquelle

$R_2'$ représente un atome d'hydrogène, un groupe alcoyle avec 1 à 3 atomes de carbone, un groupe alcoxy-carbonyle avec en tout 2 à 4 atomes de carbone, un groupe phényle ou halogénophényle, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 qtomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

6. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I dans laquelle $R_1$ possède les significations mentionnées dans la revendication 1 pour $R_1$ à l'exception de l'atome d'hydrogène et du groupe phényle, on fait réagir une pyrazine de formule générale

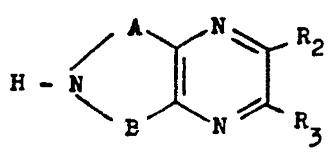

(XI)

dans laquelle

$R_2$, $R_3$, A et B sont définis comme dans la revendication 1, avec un composé de formule générale

$R_1'$—Y (XII)

dans laquelle

$R_1'$ possède les significations mentionnées dans la revendication 1 pour $R_1$ à l'exception de l'atome d'hydrogène et du groupe phényle et

Y représente un groupe partant nucléophile, dans un solvant et

si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophényle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un

composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $r_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

7. Procédé pour la préparation de nouvelles pyrazines de formule générale I selon la revendication 1 et de leurs sels d'addition d'acides, en particulier de leurs sels physiologiquement supportables avec des acides minéraux ou organiques, caractérisé en ce que, pour la préparation de composés de formule générale I dans laquelle l'un des radicaux $R_2$ ou $R_3$ représente un groupe amino et l'autre des radicaux $R_2$ ou $R_3$ représente un groupe alcoxy avec 1 à 3 atomes de carbone, on fait réagir une pyrazine de formule générale

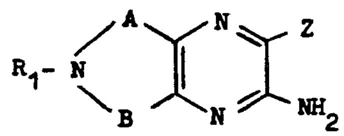

(XIII)

dans laquelle

$R_1$, A et B sont définis comme dans la revendication 1 et

Z représente un groupe partant nucléophile tel qu'un atome d'halogène, avec un composé de formule générale

$$H—R_2''$$ (XIV)

dans laquelle

$R_2''$ représente un groupe alcoxy avec 1 à 3 atomes de carbone, ou son sel alcalin dans un solvant et si on le désire ensuite on transforme un composé obtenu selon l'invention, de formule générale I dans laquelle $R_1$ représente un groupe alcanoyle avec 1 à 3 atomes de carbone éventuellement substitué par un groupe phényle ou halogénophyle, ou représente un groupe alcoxy-carbonyle avec en tout 2 à 4 atomes de carbone, ou représente un groupe aralcoxy-carbonyle avec en tout 8 à 10 atomes de carbone, au moyen d'une hydrolyse et éventuellement une décarboxylation subséquente, en un composé correspondant de formule générale I dans laquelle $R_1$ représente un atome d'hydrogène et/ou

on transforme un composé obtenu de formule générale I dans laquelle $R_1$ représente un groupe alcanoyle avec 1 à 3 atomes de carbone éventuellement substitué par un groupe phényle ou halogénophényle, ou représente un groupe alcoxycarbonyle avec en tout 2 à 4 atomes de carbone ou un groupe aralcoxy-carbonyle avec en tout 8 à 10 atomes de carbone, au moyen d'une réduction, en un composé correspondant de formule générale I dans laquelle $R_1$ représente un groupe alcoyle avec 1 à 3 atomes de carbone éventuellement substitué par un groupe phényle ou halogénophényle et/ou

on transforme un composé obtenu de formule générale I dans laquelle $R_1$ représente un groupe benzyle éventuellement substitué par un atome d'halogène, par réaction avec le chloroformiate de benzyle et ensuite avec de l'acide acétique glacial/acide bromhydrique, en un composé correspondant de formule générale I dans laquelle $R_1$ représente un atome d'hydrogène et/ou

on transforme un composé obtenu de formule générale I dans laquelle $R_2$ représente un atome d'hydrogène et $R_3$ un groupe amino, au moyen d'une halogénation, en un composé correspondant de formule générale I dans laquelle $R_2$ représente un atome d'halogène et/ou

on transforme un composé obtenu de formule générale I en son sel d'addition d'acide, en particulier en son sel physiologiquement supportable avec un acide minéral ou organique.

8. Procédé pour la préparation de nouvelles pyrazinoazépines de formule générale

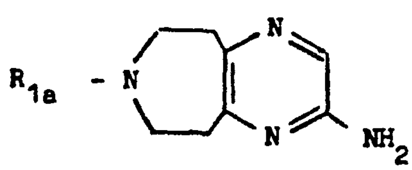

(Ia)

dans laquelle

$R_{1a}$ représente un atome d'hydrogène, un groupe alcoyle avec 1 à 6 atomes de carbone, un groupe alcényle avec 3 à 6 atomes de carbone, un groupe alcoyle avec 1 à 3 atomes de carbone qui est substitué par un groupe phényle éventuellement substitué par un atome d'halogène, ou représente un groupe cycloalcoyle avec 5 à 7 atomes de carbone, un groupe alcanoyle avec 1 à 3 atomes de carbone, un groupe

alcoxy-carbonyle avec en tout 2 à 4 atomes de carbone, un groupe benzoyle éventuellement substitué par un atome d'halogène ou représente le groupe phényle, et de leurs sels d'addition d'acides physiologiquement spportables avec des acides minéraux ou organiques, caractérisé en ce qu'on fait réagir une dicétone éventuellement non purifiée de formule générale

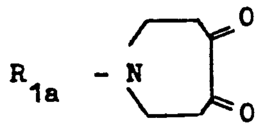

(IIa)

dans laquelle

$R_{1a}$ est défini comme au début, dans un solvant avec de la 2-amino-acétamidine et

si on le désire on transforme ensuite un composé obtenu selon l'invention de formule générale la dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'halogène, au moyen d'une hydrolyse et éventuellement d'une décarboxylation subséquente, en le composé de formule générale la dans laquelle $R_{1a}$ représente l'hydrogène et/ou

on transforme un composé obtenu de formule générale la dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'ahlogène, au moyen d'une réduction, en un composé de formule générale la dans laquelle $R_{1a}$ représente un groupe alcoyle avec 1 à 3 atomes de carbone ou un groupe benzyle éventuellement substitué par un atome d'halogène et/ou

on transforme un composé obtenu de formule générale la en ses sels d'addition d'acides physiologiquement supportables avec des acides minéraux ou organiques.

9. Procédé pour la préparation de nouvelles pyrazinoazépines de formule générale la selon la revendication 8 et de ses els d'addition d'acides physiologiquement supportables avec des acides minéraux ou organiques, caractérisé en ce qu'on réduit un pyrazine-N-oxyde éventuellement formé dans le mélange réactionnel, de formule générale

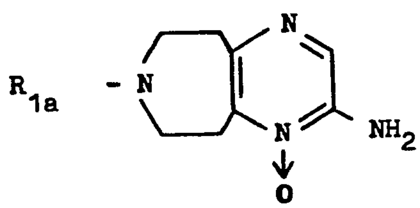

(IVa)

dans laquelle

$R_{1a}$ est défini comme dans la revendication 8, dans un solvant et

si on le désire on transforme ensuite un composé obtenu selon l'invention de formule générale la dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'halogène, au moyen d'une hydrolyse et éventuellement d'une décarboxylation subséquente, en le composé de formule générale la dans laquelle $R_{1a}$ représente l'hydrogène et/ou

on transforme un composé obtenu de formule générale la dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'halogène, au moyen d'une réduction, en un composé de formule générale la dans laquelle $R_{1a}$ représente un groupe alcoyle avec 1 à 3 atomes de carbone ou un groupe benzyle éventuellement substitué par un atome d'halogène et/ou

on transforme un composé obtenu de formule générale la en ses sels d'addition d'acides physiologiquement supportables avec des acides minéraux ou organiques.

10. Procédé pour la préparation de nouvelles pyrazinoazépines de formule générale la selon la revendication 8 et de leurs sels d'addition d'acides physiologiquement supportables avec des acides minéraux ou organiques, caractérisé en ce qu'on cyclise un composé éventuellement formé dans le mélange réactionnel, de formule générale

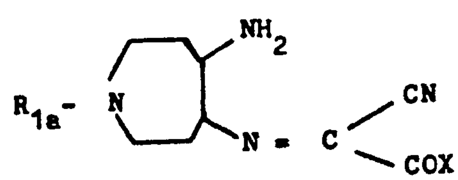

(Va)

dans laquelle

$R_{1a}$ est défini comme dans la revendication 8 et

X représente un groupe alcoxy, et on hydrolyse et décarboxyle un composé ainsi obtenu de formule générale

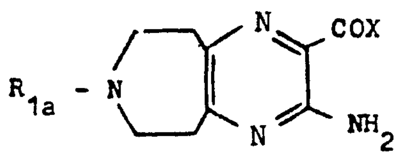

(VIa)

dans laquelle

$R_{1a}$ et X sont définis comme plus haut, dans un solvant et si on le désire on transforme ensuite un composé obtenu selon l'invention de formule générale Ia dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'halogène, au moyen d'une hydrolyse et éventuellement d'une décarboxylation subséquente, en le composé de formule générale Ia dans laquelle $R_{1a}$ représente l'hydrogène et/ou

on transforme un composé obtenu de formule générale Ia dans laquelle $R_{1a}$ représente un groupe alcanoyle avec 1 à 3 atomes de carbone, un groupe alcoxy-carbonyle avec en tout 2 à 4 atomes de carbone ou groupe benzoyle éventuellement substitué par un atome d'halogène, au moyen d'une réduction, en un composé de formule générale Ia dans laquelle $R_{1a}$ représente un groupe alcoyle avec 1 à 3 atomes de carbone ou un groupe benzyle éventuellement substitué par un atome d'halogène et/ou

on transforme un composé obtenu de formule générale Ia en ses sels d'addition d'acides physiologiquement supportables avec des acides minéraux ou organiques.

11. Procédé pour la préparation de nouvelles pyrazino-azépines de formule générale Ia selon la revendication 8 et de leurs sels d'addition d'acides physiologiquement supportables avec des acides minéraux ou organiques, caractérisé en ce qu'on fait réagir une pyrazino [2,3-d] azépine de formule générale

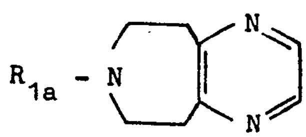

(VIIa)

dans laquelle

$R_{1a}$ est défini comme à la revendication 8, avec un amidure alcalin, dans un solvant et

si on le désire on transforme ensuite un composé obtenu selon l'invention de formule générale Ia dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'halogène, au moyen d'une hydrolyse et éventuellement d'une décarboxylation subséquente, en le composé de formule générale Ia dans laquelle $R_{1a}$ représente l'hydrogène et/ou

on transforme un composé obtenu de formule générale Ia dans laquelle $R_{1a}$ représente un groupe alcanoyle avec 1 à 3 atomes de carbone, un groupe alcoxy-carbonyle avec en tout 2 à 4 atomes de carbone ou un groupe benzoyle éventuellement substitué par un atome d'halogène, au moyen d'une réduction, en un composé de formule générale Ia dans laquelle $R_{1a}$ représente un groupe alcoyle avec 1 à 3 atomes de carbone ou un groupe benzyle éventuellement substitué par un atome d'halogène et/ou

on transforme un composé obtenu de formule générale Ia en ses sels d'addition d'acides physiologiquement supportables avec des acides minéraux ou organiques.

12. Procédé selon la revendication 5, caractérisé en ce que l'on cyclise un composé formé dans le mélange réactionnel de formule générale

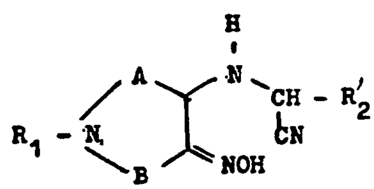

(X)

dans laquelle

$R_1$, $R_2$, A et B sont définis comme dans la revendication 5, en présence d'un agent de condensation acide.